# EUROPEAN PATENT APPLICATION

(11) **EP 2 578 674 A1**
(43) Date of publication of application: **10.04.2013**
(21) Application number: 11786731.7
(22) Date of filing: 26.05.2011
(51) Int. Cl.: C12M 1/00, C12M 1/42, G01N 37/00, C12Q 1/68

(54) **BIOLOGICAL-SAMPLE AFFIXING DEVICE**

(30) Priority: 30.06.2010 JP 2010150523; 26.05.2010 JP 2010119995; 26.05.2010 JP 2010119984
(71) Applicant: Tosoh Corporation, Yamaguchi 746-8501 (JP)
(72) Inventor: MORIMOTO Atsushi, Ayase-shi Kanagawa 252-1123 (JP); FUTAMI Toru, Ayase-shi Kanagawa 252-1123 (JP); MOGAMI Toshifumi, Ayase-shi Kanagawa 252-1123 (JP)
(74) Representative: Pautex Schneider, Nicole Véronique
(86) International application number: PCT/JP2011/062141
(87) International publication number: WO 2011/149032

(57) **Abstract**

A biological sample immobilizing apparatus is provided, comprising a holding unit which holds a biological sample, a pair of electrodes which allows a dielectrophoretic force to act on the biological sample to move the biological sample to the holding unit, and a power source which applies an AC voltage to the pair of electrodes. Accordingly, a plurality of components contained in the biological sample can be conveniently separated into individuals one by one and respective genes thereof can be analyzed one by one quickly and simultaneously, while providing a high density and being miniaturized.

## Description

### TECHNICAL FIELD

The present invention relates to a biological sample immobilizing apparatus (an apparatus for immobilizing a biological sample).

### BACKGROUND ART

Genetic factors and environmental factors are involved in various diseases. Regarding certain diseases such as congenital metabolic disorder, cancer, diabetes, hypertonia, Alzheimer's disease, autoimmune disease, obesity, and alcoholism, the involvement of a genetic factor, i.e. the involvement of a gene, is responsible for the disease at a large proportion. In recent years, due to the rapid progress in the molecular biology, the involvement of a genetic factor is concretely elucidated in relation to various diseases. Also, attention is focused on the early detection and the medical treatment of a disease on the basis of the analysis that targets genes.
Further, for example, it is known that in the case of a certain type of disease, cells having a property specific to the disease exist, such as the following: cancer cells having different properties exist even in the cancer of the same tissue; and the degree of the progress and malignancy, the possibility of metastasis, the effect of an agent for medical treatment, or the prognosis differ depending on the property of the cancer cell. Therefore, it is considered that important information or the like for selecting a medical treatment method or an agent used for the medical treatment can be obtained, for example, by analyzing the property of the cell relevant to a disease such as the cancer cell and classifying the cancer on the basis of the obtained result.
Further, for example, it is also considered that whether or not a cell becomes cancerous is analyzed at an extremely early stage by reading out a mutation on a specific gene in the cell. In fact, the analysis and the identification of so-called oncogene (cancer gene), which is involved in the canceration (oncogenesis) of a cell and the abnormal proliferation of a cancer cell, are in progress. Further, the identification of a cancer suppressor gene, of which mutation or decreased expression results in the canceration, is also in progress. Until now, Rb gene of retinoblastoma, p53 gene and APC gene of colon cancer, WT1 gene of Wilms tumor, and so forth have been reported as the cancer suppressor genes.
As described above, a large number of genes relevant to cancers are known. In many cases, the analysis of the gene is performed by utilizing, for example, the PCR method by which the target area in the gene can be amplified (Patent Documents 1 to 3), the fluorescence *in situ* hybridization (FISH) method by which a mutation of the gene is found out by visualizing the mutation via the labeling of chromosomal DNA, and so forth.

### PRIOR ART REFERENCES

### Patent documents

- Patent Document 1:: United States Patent No. 4,683,195;
- Patent Document 2:: United States Patent No. 4,683,202;
- Patent Document 3:: United States Patent No. 4,800,159;
- Patent Document 4:: JP 3723882 B2;
- Patent Document 5:: JP 2007-295912 A;
- Patent Document 6:: JP 3097932 B2.

### SUMMARY OF THE INVENTION

### OBJECT TO BE ACHIEVED BY THE INVENTION

In order to analyze the property of a cell relevant to a disease or read out a mutation on a specific gene in the cell, it is conceived that an affected tissue, a body fluid, or the like of a subject is sampled to analyze cells contained therein. For example, in the case of a cancer cell, it is conceived that a cancer tissue is sampled to analyze the property of cells contained in the tissue. Further, for example, because the cancer cell dissociated from a primary tumor mass can cause systemic metastasis while being spread into blood vessel, lymphatic vessel, or abdominal cavity, it is also conceived that cells contained in the body fluid are analyzed.

When the analysis is performed as described above, the most important is simply that a biological sample such as cells contained in a tissue, a body fluid, or the like is separated into individuals to analyze the properties of the individuals or a specific gene in the individuals. Any conventional example has been scarcely known in relation to the method for individually analyzing a biological sample (for example, cells), but only the following report has been made. That is, each of lymphocytes in blood was introduced into one microwell one by one, these lymphocytes were stimulated with an antigen, antigen-specific lymphocytes with a low existence probability (not more than 0.1%), which responded to the antigen, were detected, and an antigen-specific antibody gene was cloned from the antigen-specific lymphocyte to produce a human type monoclonal antibody in a large amount from the gene (the above-described Patent Document 4).
In the techniques for analyzing a gene which has been hitherto frequently used such as the above-described PCR method (Patent Documents 1 to 3) and the above-described FISH method (Patent Document 4), cancer cells, which are present in the same tissue and which have different properties, are analyzed in a manner such that the difference among individuals is averaged, rather than that individuals are analyzed. Therefore, a problem arises such that any information about the individuals of a biological sample (for example, individual cells) cannot be obtained from the analysis result obtained by the conventional method.
In Patent Document 4, the cells are immobilized one by one to a plurality of microwells formed in an array form to perform the analysis. However, the actual operation is performed by repeating such a procedure that a cell suspension is added to microwells each having an inner diameter and a depth which are one time to two times the diameter of lymphocyte to be immobilized, and the cells outside the wells are washed out after waiting for the sedimentation of the cells in the wells. Accordingly, in the case of the method described in the above-described Patent Document 4, a problem arises such that the time required to wait for the sedimentation of cells in accordance with the gravity is about 5 minutes, which is long, repeating the procedure of washing after waiting for the sedimentation of cells is time-consuming, and the cells which are not introduced into the wells are lost during the waiting and hence information cannot be obtained from all of the cells subjected to the analysis.

Patent Document 5 discloses such a technique that an AC voltage is applied between electrodes which are provided at upward and downward positions with respect to a plurality of through-holes formed in an array form to allow the dielectrophoretic force to act, and thereby cells are immobilized one by one to the through-holes to perform the cell fusion. In this technique, the sizes of the two cells to be fused and the size of the through-hole are set to be in a certain relationship, and thereby the cells can be fused efficiently. However, a problem arises such that it is impossible to analyze what gene sequence the individual fused cell has. The dielectrophoretic force is utilized not only for the immobilization of cells as described in Patent Document 5 but also for the analysis of proteins contained in a sample on chromatography as described in Patent Document 6.

According to the above, an object of the present invention is to provide an analysis method by which a biological sample is conveniently separated into individuals, the individuals are immobilized one by one, and then the properties of the individuals or a specific gene in the individuals can be quickly analyzed.

### MEANS FOR ACHIEVING THE OBJECT

As a result of diligent investigations performed by the inventors of the present invention in order to achieve the above-described object, the present invention has been completed. That is, the present invention is a biological sample immobilizing apparatus comprising a holding unit for holding a biological sample, a pair of electrodes for allowing a dielectrophoretic force to act on the biological sample to move the biological sample to the holding unit, and a power source for applying an AC voltage to the pair of electrodes. In another aspect, the present invention is a gene extracting apparatus comprising a holding unit for holding a biological sample, a pair of electrodes for allowing a dielectrophoretic force to act on the biological sample to move the biological sample to the holding unit, a power source for applying an AC voltage to the pair of electrodes, and disrupting means for disrupting the biological sample immobilized to the holding unit. In still another aspect, the present invention is a gene analysis apparatus comprising a holding unit for holding a biological sample, a pair of electrodes for allowing a dielectrophoretic force to act on the biological sample to move the biological sample to the holding unit, a power source for applying an AC voltage to the pair of electrodes, disrupting means for disrupting the biological sample immobilized to the holding unit, and detecting means for detecting a gene extracted from the disrupted biological sample. Also, the present invention is a gene analysis method comprising allowing a dielectrophoretic force to act on a biological sample, moving and immobilizing the biological sample to a holding unit, disrupting the immobilized biological sample, and detecting a gene extracted from the disrupted biological sample. The present invention will be explained in detail below.

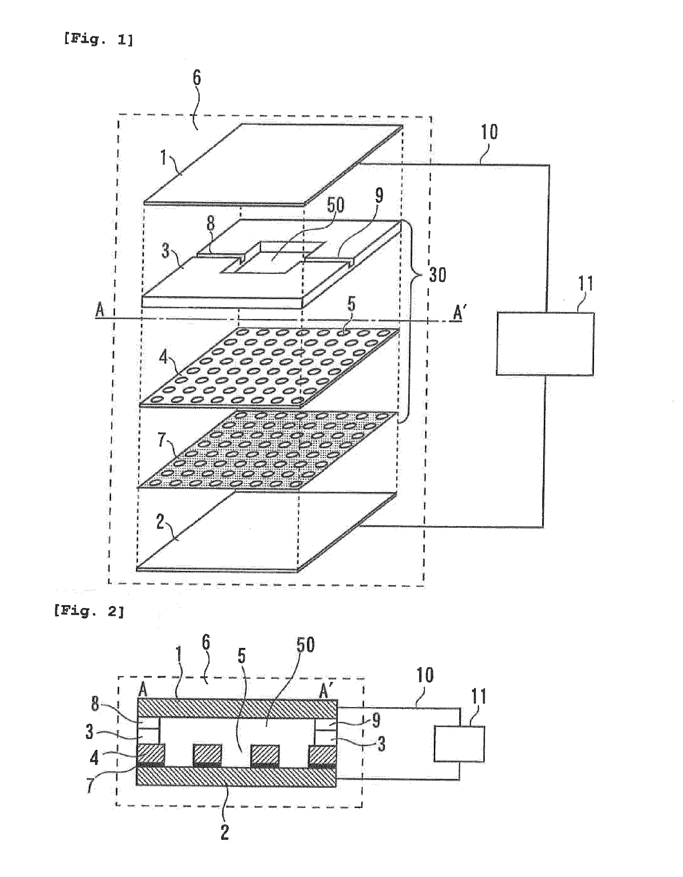

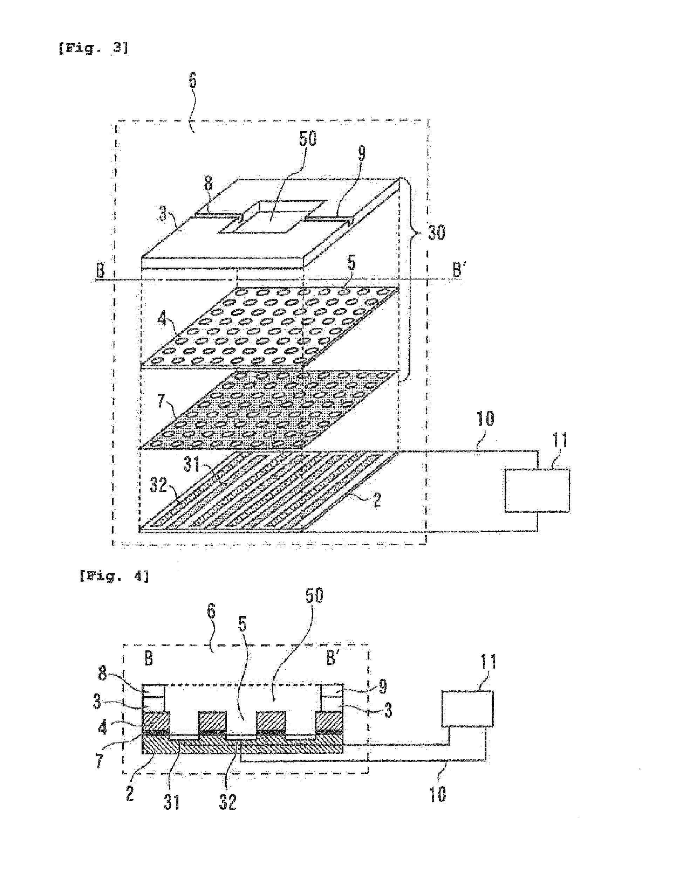

The biological sample immobilizing apparatus of the present invention (hereinafter referred to as "immobilizing apparatus" in some cases) has a holding unit for holding a biological sample. The holding unit is not particularly limited as long as the holding unit has a portion (hereinafter referred to as "holding portion" in some cases) which has such a size (dimensions) and such a shape that one individual of the biological sample such as a cell can be immobilized. For example, a flat plate composed of one member or constructed by staking a plurality of members, wherein a through-hole is provided as the holding portion, can be exemplified. The immobilizing apparatus of the present invention is provided with a pair of electrodes in order that the dielectrophoretic force is allowed to act on the biological sample by means of the electrodes and thereby the biological sample is moved to the holding portion of the holding unit. Therefore, the holding unit and the electrodes are arranged so that the electric flux line (line of electric force) which is generated between the electrodes when the voltage is applied between the pair of electrodes passes through the holding portion. For example, when the holding portion is provided as such a through-hole as described above, as shown in Fig. 1, an arrangement such that one end of the through-hole is closed by one electrode to form a bottom portion, and the other electrode is arranged so as to be opposed to the electrode which closes the holding portion (through-hole) can be exemplified. Also, as shown in Fig. 3, an arrangement such that two or more holding portions (through-holes) are provided, one end of each of some of the holding portions (through-holes) is closed by one electrode to form a bottom portion, and one end of each of the remaining holding portion(s) (through-hole(s)) is closed by the other electrode to form a bottom portion can be exemplified. In order that the electric flux line passes through the holding portions, for example, the holding unit is composed of a plurality of members including an insulative member as shown in Fig. 1, or one member or a plurality of members constituting the holding unit is/are entirely composed of insulator(s), so that the electricity does not conduct to any part other than the electrode.

In order that an AC voltage is applied to the pair of electrodes thereby to allow the dielectrophoretic force that moves the biological sample to the holding portion of the holding unit to act, the immobilizing apparatus of the present invention is provided with a power source. The power source is not particularly limited as long as it can apply the AC voltage to the pair of electrodes.

The biological sample, which is to be immobilized to the holding portion of the holding unit, is supplied as a conductive biological sample suspension containing the biological sample. For this purpose, an accommodating unit for accommodating the biological sample suspension is provided, and an example of the accommodating unit can include a liquid reservoir that is communicated with the holding portion. More specifically, as shown in Fig. 1, an arrangement such that a spacer (frame) that provides the portion for accommodating the biological sample suspension is provided above the holding unit so that the biological sample suspension accommodated in the accommodating unit can flow into the holding portion of the holding unit can be exemplified. As shown in Fig. 1, when one end of the holding portion (through-hole) is closed by one electrode to form the bottom portion and the other electrode is arranged to be opposed to the electrode for closing the holding portion (through-hole), then the accommodating unit is arranged between the holding unit and the other electrode and filled with the biological sample suspension, and thereby the electric flux line passes between the pair of electrodes via the holding portion (through-hole).
Also, as shown in Fig. 3, when two or more of the holding portions (through-holes) are provided to provide such an arrangement that one end of each of some of the holding portions (through-holes) is closed by one electrode to form the bottom portion and one end of each of the remaining holding portion(s) (through-hole(s)) is closed by the other electrode to form the bottom portion, then the accommodating unit is arranged above the holding portions, and thereby the electric flux line passes between the pair of electrodes via the holding portions, i.e., between one electrode provided as the bottom surface of a certain holding portion and the other electrode provided as the bottom surface of another holding portion.

The biological sample immobilizing apparatus according to the present invention can be grasped from another aspect. Specifically, the biological sample immobilizing apparatus according to the present invention comprises a holding unit which has a holding portion for holding a biological sample; a power source which is capable of applying an AC voltage; and a pair of electrodes which allows a dielectrophoretic force to act on the biological sample in a given dielectrophoretic space by means of the AC voltage applied from the power source thereby to move the biological sample to the holding portion. Further, each of the pair of electrodes is arranged so that the dielectrophoretic force can act on the biological sample in the given dielectrophoretic space from the side of a common electrode installation position positioned on a holding unit side with respect to the given dielectrophoretic space.

In the biological sample immobilizing apparatus configured as described above, the above-described given dielectrophoretic space refers to the space in which the dielectrophoretic force can be allowed to act on the biological sample by means of the electric flux line generated between the pair of electrodes. Therefore, the given dielectrophoretic space can also include the internal space of the holding portion to which the biological sample is finally moved so as to be immobilized. The given dielectrophoretic space can also include other spaces connected to this internal space so that the biological sample is movable (for example, a space corresponding to the above-described "accommodating unit"). As for the holding portion possessed by the holding unit, the number of the holding portion can be one or can be two or more as long as the biological sample can be immobilized to the holding portion(s) in accordance with the action of the dielectrophoretic force. Therefore, in such a biological sample immobilizing apparatus according to the present invention, the biological sample is moved to the holding portion(s) possessed by the holding unit from the given dielectrophoretic space in accordance with the dielectrophoretic force acting between the pair of electrodes, and then held therein. In this case, each of the pair of electrodes is arranged so that the dielectrophoretic force can be allowed to act on the biological sample from the common electrode installation position positioned on the side of the holding unit with respect to the given dielectrophoretic space, in other words, each of the pair of electrodes is arranged so as not to be in such a state that the given dielectrophoretic space is interposed by the pair of electrodes. This fact results in that in the biological sample immobilizing apparatus according to the present invention, the given dielectrophoretic space, which is the physical space, is not allowed to exist between the electrodes, and thereby, it is possible to shorten the distance between the electrodes as much as possible. Therefore, regarding the pair of electrodes, even when the voltage applied between the electrodes is relatively low, the dielectrophoretic force acting on the biological sample can be retained to be large. In other words, the biological sample can be immobilized in accordance with the efficient dielectrophoresis.

In the above-described biological sample immobilizing apparatus, when the holding unit has at least two of the holding portions, the electrode installation positions, at which the respective electrodes of the pair of electrodes are arranged, can be configured so as to be positioned on a common flat surface which defines the respective internal spaces of the at least two holding portions possessed by the holding unit. When the electrode installation positions are configured as described above, then a pair of electrodes is arranged to each of the holding portions, and the relative positions of the electrodes with respect to the respective holding portions can be made approximately uniform. As a result, the biological sample can be uniformly held in the plurality of holding portions, and the efficient holding of the biological sample can be realized.

In this context, in the above-described biological sample immobilizing apparatus, one electrode of the pair of electrodes can be arranged to be capable of making contact with the biological sample in some holding portion(s) of the at least two holding portions, and the other electrode of the pair of electrodes can be arranged to be capable of making contact with the biological sample in the remaining holding portion(s) of the at least two holding portions. That is, by arranging the pair of electrodes on the above-described common flat surface so that the electrodes can make contact with the biological sample in the respective holding portions, the biological sample can be held by the holding portions more reliably when the voltage is applied.

In the above-described biological sample immobilizing apparatus, when the holding portions each have an opening through which the biological sample enters from the given dielectrophoretic space, then the one electrode can be configured so as to be arranged at the bottom portion of the holding portion opposed to the opening in the some holding portion(s), and the other electrode can be configured so as to be arranged at the bottom portion of the holding portion opposed to the opening in the remaining holding portion(s). When the electrodes are configured as described above, the gravity acting on the biological sample can be also utilized when the biological sample is held in the holding portion, and hence, it is possible to realize the more efficient immobilization of the biological sample.

In this context, in the above-described biological sample immobilizing apparatus, it is also allowable to adopt such a configuration that the pair of electrodes comprises a first electrode which has a plurality of small electrode portions and which is constructed by connecting the small electrode portions, and a second electrode which is independent from the first electrode, which has a plurality of small electrode portions, and which is constructed by connecting the small electrode portions; and at least some of the plurality of small electrode portions of the first electrode and at least some of the plurality of small electrode portions of the second electrode are alternately arranged while keeping a given distance between the electrodes on the common flat surface. In another way, in the concerning biological sample immobilizing apparatus, it is also allowable to adopt such a configuration that the pair of electrodes comprises a first electrode which has a continuous line form and a second electrode which is independent from the first electrode and which has a continuous line form; and the first electrode and the second electrode are arranged on the common flat surface while keeping a given distance between the electrodes in a length direction between both of the electrodes. These configurations are referred to by way of example in every sense, and it is not intended to limit the present invention to any one of these configurations.

In the case of the former configuration, the small electrode portions constituting the first electrode and the small electrode portions constituting the second electrode are alternately arranged while mutually keeping the given distance between the electrodes. The given distance between the electrodes, which is referred to herein, refers to the distance which is sufficient to generate the dielectrophoresis of the biological sample as the target of the immobilization. Therefore, by adopting such a configuration of arrangement, the dielectrophoretic force, which is generated between the first electrode and the second electrode, can be alternately generated at the small electrode portion at the side of the first electrode and the small electrode portion at the side of the second electrode. Therefore, as the repetitions of the respective small electrode portions are arranged more densely between the first electrode and the second electrode, the dielectrophoretic force can be allowed to act on more of the biological samples, and the biological samples can be efficiently held in the holding portions. Further, by arranging the repetitions densely as described above, it is possible to realize the effective generation of the dielectrophoretic force while further decreasing the voltage applied between the electrodes. An example of such a configuration can include a comb-shaped electrode configuration shown in Fig. 3.

On the other hand, in the case of the latter configuration, each of the pair of electrodes is formed in the line form, and the distance between the electrodes is kept to be the given distance between the electrodes in the extending direction of the electrodes. The given distance between the electrodes, which is referred to herein, refers to the distance which is sufficient to generate the dielectrophoresis of the biological sample as the target of the immobilization. Therefore, by adopting such a configuration of arrangement, the dielectrophoretic force, which is required to hold the biological sample in the holding portion, can be generated between the "line" constituting the first electrode and the "line" constituting the second electrode. In particular, when the distance between the electrodes is kept to be the given distance between the electrodes over the entire length of the first electrode and the entire length of the second electrode, then the biological sample can be immobilized at any position between the electrodes by appropriately positioning the electrodes with respect to the holding portion, and the degree of freedom of the design is enhanced for the biological sample immobilizing apparatus. An example of such a configuration can include such a configuration that the first electrode and the second electrode each are arranged in a continuous curved line form or in a continuous polygonal line form on the common flat surface.

In this context, in the above-described biological sample immobilizing apparatus, it is also allowable to adopt such a configuration that one end of the holding unit is opened so that the biological sample immobilized to the holding unit can be collected (sampled). That is, because the pair of electrodes is arranged one-sidedly with respect to the given dielectrophoretic space as described above, as a result, it is possible to prevent the immobilized biological sample from being in such a state that the immobilized biological sample is interposed between the electrodes. As a result, it is easy to access the biological sample after the immobilization. Hence, it is possible to easily and appropriately realize not only the collection (sampling) of the biological sample but also the supply of a solvent to the biological sample, the observation of the immobilized biological sample, and so forth.

According to such an immobilizing apparatus of the present invention as described above, the electric flux line passes between the electrodes when the AC voltage is applied between the pair of electrodes, the dielectrophoretic force is generated for the biological sample contained in the biological sample suspension in the given dielectrophoretic space such as the accommodating unit and the internal space of the holding portion, and hence, the biological sample is moved along the electric flux line to the holding portion of the holding unit. Accordingly, the biological sample is moved to one holding portion. The holding portion has a size (dimensions) and a shape for immobilizing one individual of the biological sample, and hence, two individuals of the biological sample are not immobilized to one identical holding portion. The biological sample, which was moved to the holding portion, is immobilized to the holding portion during the period while the AC voltage is applied. In order that the biological sample is immobilized to the holding portion even after the stop of the application of the AC voltage to the electrodes, it is preferable that a substance that has the affinity for the biological sample intended to be immobilized is immobilized to the holding portion beforehand.

A gene extracting apparatus of the present invention (hereinafter referred to as "extracting apparatus" in some cases) comprises a disrupting means for disrupting the biological sample immobilized to the holding unit, in addition to the above-described biological sample immobilizing apparatus. The holding unit, the pair of electrodes, or the power source is similar to that of the immobilizing apparatus. The disrupting means, which is provided for the extracting apparatus, is not particularly limited as long as it can disrupt the biological sample and thereby the nucleic acid contained in the biological sample can be eluted to the outside of the biological sample. For example, it is possible to exemplify a means consisting of a pair of electrodes and a power source for applying a DC voltage or an AC voltage having a low frequency of about 1 Hz to the electrodes, wherein the voltage is applied to the biological sample immobilized to the holding portion of the holding unit. When the biological sample is a cell having no cell wall, the cell can be disrupted by applying a voltage of about 1 V to the cell membrane. The electrodes for allowing the dielectrophoretic force to act on the biological sample thereby to move the biological sample to the holding unit can be used also as the pair of electrodes of the disrupting means. Further, for example, it is possible to exemplify a heating means for heating the biological sample immobilized to the holding portion of the holding unit. The biological sample can be disrupted by allowing the biological sample to be under a temperature condition of 90°C for about several minutes. Further, for example, it is possible to exemplify an ultrasonic wave generating means for vibrating the biological sample immobilized to the holding portion of the holding unit.
The biological sample can be disrupted by applying the vibration of 20 to 40 kHz continuously or intermittently at an output of 100 to 200 W. It is not needed that each of the means for applying the voltage, the heating means, and the ultrasonic wave generating means, which have been exemplified by way of example, is utilized exclusively, and for example, it is also possible to utilize both of the heating means and the ultrasonic wave generating means.

As described above, the holding unit has the holding portion which has such a size (dimensions) and such a shape that one individual of the biological sample such as a cell can be immobilized. As a result, one individual of the biological sample is immobilized to one holding portion, and hence, it is also possible to obtain the nucleic acid contained in one individual of the biological sample by disrupting the immobilized biological sample in the holding portion.

A gene analysis apparatus of the present invention (hereinafter referred to as "analysis apparatus" in some cases) comprises detecting means for detecting the gene extracted from the disrupted biological sample, in addition to the above-described gene extracting apparatus. The holding unit, the pair of electrodes, the power source, or the disrupting means is similar to that of the immobilizing apparatus or that of the extracting apparatus.

The detecting means, which is provided for the analysis apparatus, is a means for analyzing the property or the like of the cell immobilized to the holding portion of the holding unit by detecting the nucleic acid eluted to the outside of the biological sample by disrupting the biological sample. For example, it is possible to exemplify a known optical detecting means for detecting the light emission or the fluorescence intensity when a nucleic acid probe labeled with a light-emitting substance, a fluorescent substance, or the like is used, a known optical detecting means for detecting the absorbance or the turbidity, an RI detecting means, an optical microscope for visually recognizing such an optical change by means of the visual observation, a magnifying means for magnifying and observing the holding portion in order to detect the turbidity change or the like by means of the visual observation, and so forth. When the optical detecting means is utilized as the detecting means, if referring, for example, to an exemplary case where an exciting light beam or the like is radiated and a fluorescence or the like coming from the holding portion is received and detected, it is allowable to adopt any one of an embodiment in which the radiation of the exciting light beam or the like and the receiving of the fluorescence or the like are performed in the same direction with respect to the holding portion of the holding unit (for example, the exciting light beam or the like is radiated from a direction upward from the holding portion and the light is received in the direction upward from the holding portion) and an embodiment in which the radiation of an exciting light beam or the like and the receiving of a fluorescence or the like are performed on different sides (for example, the exciting light beam or the like is radiated from a direction upward from the holding portion and the light is received in a direction downward from the holding portion). In any case, for example, a member through which the exciting light beam, the fluorescence, and the like can pass is selected as the member for constituting the substrate on which the electrodes are provided or the member for constituting the accommodating unit as described later on.

The detecting means is not limited to such a means that the nucleic acid eluted from the biological sample to the outside thereof is detected as it is, and the detecting means can be, for example, such a means that the eluted nucleic acid is detected after the amplification. For the amplification of the nucleic acid, it is possible to utilize a method known *per* se such as the PCR method, the LAMP method, the RT-PCR method, the NASBA method, the TMA method, or the TRC method. When an amplification in which the temperature cycling is required to carry out the amplification as in the PCR method is performed, a temperature-regulating means is added to the detecting means. By contrast, in the case of an amplification method that can be carried out at a constant temperature such as the TRC method, it is sufficient that, for example, a temperature-regulating means for regulating the temperature to the constant temperature is provided, or the analysis apparatus itself is put in a space that is temperature-regulated at the constant temperature. In the temperature-regulating means for carrying out the PCR method, the temperature of the holding portion is raised to about 90°C in the temperature cycling. Therefore, when the heating means is utilized as the above-described disrupting means, the heating means can be commonly used for the both of the temperature-regulating means and the disrupting means.

It is also possible to exemplify an embodiment in which a nucleic acid probe, a color reagent, or the like is utilized in order to generate the fluorescence or the change in the turbidity when a sequence specifically found in a specified gene is used as a target to amplify the sequence by means of the PCR method or the like. When the presence of the target gene or the like can be known by means of the visual observation without using such a detecting means as described above, for example, by performing the nucleic acid amplification or by utilizing such a probe or reagent after performing the nucleic acid amplification, it is possible to analyze the gene by using the above-described extracting apparatus.

When the holding unit of the analysis apparatus of the present invention is provided with a plurality of holding portions, and the analysis apparatus can analyze the respective genes extracted from two or more individuals of the biological samples individually by using the optical detecting means, then it is preferable to provide, for example, a light shielding member as shown in Fig. 1 in order that an optical signal coming from a certain holding portion is not affected by an optical signal coming from another holding portion. When the plurality of holding portions are provided for the holding unit, the same number of the optical detecting means as the number of the holding portions can be provided. Or, in order to simplify the apparatus configuration and thereby improve the maintenance and operation performance, it is preferable to adopt such a configuration that a smaller number of the optical detecting means than the number of the holding portions is/are provided, and the optical detecting means is/are driven by appropriate actuator(s) to perform the scanning for the respective holding portions. When the plurality of holding portions are provided for the holding unit, it is preferable to further provide such a configuration that position information or the like for identifying which one of the plurality of holding portions provided for the holding unit generated a certain optical signal, in order to easily obtain the amplified nucleic acid or the like from the specified holding portion of the plurality of holding portions thereafter.

The present invention also provides a gene analysis method (hereinafter referred to as "analysis method" in some cases) comprising allowing a dielectrophoretic force to act on a biological sample, moving the biological sample to immobilize the biological sample to a holding unit, disrupting the immobilized biological sample, and detecting a gene extracted from the disrupted biological sample. When the nucleic acid eluted from the disrupted biological sample is detected after amplified by the PCR method, the biological sample is firstly immobilized to the holding portion of the holding unit. After that, for example, a reaction solution containing primers, enzyme, substrates for enzyme, or the like for causing the PCR reaction is fed to the accommodating unit described later on, and the solution present in the holding portion communicated with the accommodating unit (solution used to suspend the biological cell) is replaced with the reaction solution.
In the PCR reaction, the temperature is raised to about 90°C for the gene eluted from the biological sample immobilized to the holding portion (through-hole). During this process, the thermal convection can be caused at the inside of the holding portion, the gene derived from the biological sample in the holding portion (through-hole) can be diffused to the outside of the holding portion (through-hole), and the gene can contaminate the biological sample suspension in the adjacent holding portion (through-hole). Therefore, it is preferable that silicon oil or the like is added dropwise to the holding portion at the stage of completion of the solution replacement and a temperature-responsive high molecular weight compound is added into the PCR reaction solution thereby to suppress such a thermal convection. If it is intended that the biological sample is suspended in the reaction solution for the PCR reaction to move the biological sample to the holding portion by means of the dielectrophoresis, then an overcurrent is provided when the voltage is applied, due to various electrolytes contained in the reaction solution for the PCR reaction, the thermal convection is caused by the heat generation, and hence, it is difficult to move and immobilize the biological sample to the holding portion. Therefore, it is preferable to perform such a solution replacement. After performing the sealing, the above-described disruption is performed and then the detection of the nucleic acid is performed.

The present invention can be also grasped from still another aspect. The biological sample immobilizing apparatus according to the present invention (hereinafter referred to as "immobilizing apparatus" in some cases) is configured as a biological sample immobilizing apparatus having a plurality of holding portions each of which holds a biological sample to detect light emitted from a substance that indicates the presence of a component constructing the biological sample, wherein the biological sample immobilizing apparatus comprises a flat plate substrate and a holding unit which is arranged on the substrate and formed with the plurality of holding portions, wherein the holding unit has at least an insulator film and a light shielding film which is provided between the insulator film and the substrate, and wherein the holding portion is opened on an upper surface of the holding unit and extends to (arrives at) the substrate via the insulator film and the light shielding film.

According to this configuration, due to the light shielding film provided for the holding unit, it is possible to reduce a light noise such as the background noise resulting from the autofluorescence of the insulator film itself and the crosstalk noise resulting from the leakage light from the adjacent holding portion, and hence, it is possible to detect only the light emitted from the observation objective substance present in each of the holding portions at a high sensitivity and a high accuracy. Because it is possible to perform the highly sensitive and highly accurate detection, it is also possible to expect the effect of shortening the detection time.

In this context, it is preferable that the structure of the present invention further comprises an accommodating unit for accommodating a suspension containing the biological sample above the holding unit, wherein the holding portion is provided so as to be communicated with the accommodating unit. Accordingly, the biological sample can be easily introduced into each of the holding portions. As the method for introducing the biological sample into the holding portion, the spontaneous sedimentation (gravity) can be utilized, or the dielectrophoretic force can be utilized. The method utilizing the dielectrophoretic is more preferred, because the biological sample can be introduced into a large number of the holding portions within an extremely short period of time of about several seconds.

In order to allow the dielectrophoretic force to act on the biological sample, it is sufficient that the AC electric field is applied so that the electric flux lines are concentrated on the holding portion in a state where the accommodating unit and the holding portion are filled with the suspension. As a configuration to apply such an AC electric field, for example, it is possible to adopt such a configuration that a pair of electrodes arranged at respective positions corresponding to the mutually different holding portions is provided on the holding unit side of the surface of the substrate, and the holding portion extends from the upper surface of the holding unit to the electrodes on the substrate. It is also possible to adopt such a configuration that a first electrode arranged at a position corresponding to the holding portion is provided on the holding unit side of the surface of the substrate, the holding portion extends from the upper surface of the holding unit to the first electrode on the substrate, and a second electrode is provided on a side opposite to the first electrode with the holding unit and the accommodating unit intervening therebetween. In the case of any configuration, the biological sample contained in the suspension can be introduced into the holding portion by applying the AC voltage having a given waveform between the two electrodes.

In this context, the light shielding film can be provided at a portion other than the plurality of holding portions of the boundary surface between the insulator film and the substrate. Preferably, the light shielding film can be provided on the entire portion other than the plurality of holding portions of the boundary surface between the insulator film and the substrate. However, when the light shielding film composed of a metallic film is used in a configuration in which the pair of electrodes is provided on the substrate, it is preferable that the light shielding film is provided only on the electrode at the portion other than the holding portions in order to avoid a short circuit between the electrodes.

The present invention will be explained in further detail below with reference to the drawings. In the immobilizing apparatus of the present invention exemplarily shown in Fig. 1, a main body 6 of the apparatus is composed of a lower electrode substrate 2 (one of a pair of electrodes), an accommodating unit 50 which is constructed by being surrounded by a spacer 3, a holding unit which is composed of a flat plate insulator 4 provided with through-holes 5 as holding portions and a light shielding member 7 arranged between the insulator and the lower electrode substrate 2, and an upper electrode substrate 1 (the other of the pair of electrodes). In this configuration, the upper electrode substrate 1 is brought in close contact with the upper surface of the spacer. When the interior of the spacer is filled with a biological sample suspension, then electric flux lines pass to the lower electrode substrate 2 via the through-holes (holding portions) 5, and the upper electrode substrate 1 also plays a role as an upper lid to cover the upper surface of the spacer thereby to prevent the scatter or evaporation of the biological sample suspension. Therefore, in the immobilizing apparatus shown in Fig. 1, the internal spaces of the accommodating unit 50 and the holding portions (through-holes) 5 correspond to a given dielectrophoretic space of the present invention.

The holding portions (through-holes) provided for the insulator 4 and the light shielding member 7, which constitute the holding unit, provide the same size (dimensions) and the same shape (circular shape), and the insulator 4 and the light shielding member 7 are stacked so that the respective through-holes are coincident with each other. One end of each of the holding portions (through-holes) of the holding unit is closed by the lower electrode substrate 2, and the biological sample can be in contact with this electrode. For example, when the accommodating unit 50 has a hermetically sealable box form and the specific gravity of the suspension containing the biological sample is not less than the specific gravity of the biological sample so that the biological sample floats in the upward direction in the suspension (even when the specific gravity of the biological sample is small, it is easy to allow the specific gravity of the suspension to be not less than the specific gravity of the biological sample), then the configuration shown in Fig. 1 can be inverted upside down, i.e., the lower electrode substrate 2 can be disposed at an upward position, and the upper electrode substrate 1 can be disposed at a downward position. However, in view of the fact that the gravity can also be utilized for the operation of the biological sample and in view of the fractional recovery of the biological sample after the immobilization and the fractional recovery of the gene eluted from the biological sample by means of the extraction, it is especially preferred that the holding unit is positioned below the accommodating unit 50 and the portion that closes the upper portion of the spacer as the accommodating unit 50 (upper electrode substrate 1 shown in Fig. 1) is made removable as exemplarily shown in Fig. 1, in order that such an operation can be carried out by means of extremely simple operation such as the suction performed from an upward position.

The biological sample of the present invention is not limited as long as the biological sample is a dielectric substance movable in accordance with the dielectrophoresis and containing the nucleic acid such as cells derived from living bodies such as animals and plants and microorganisms. Specific examples can include, for example, cells (living cells) contained in blood, lymph, cerebrospinal fluid, expectoration, urine, or feces; microorganisms, viruses, or protozoa present in the body or in the environment; and cultured cells. It is sufficient that the biological sample suspension, which is fed to the above-exemplified apparatus, is a solution in a state where such a biological sample as described above is suspended so as to be movable in accordance with the dielectrophoresis, and examples thereof can include a solution obtained by suspending the biological sample to be analyzed, for example, in an aqueous solution of a sugar such as mannitol, glucose, or sucrose, or in an aqueous solution obtained by adding an electrolyte such as calcium chloride or magnesium chloride, or a protein such as BSA (bovine serum albumin) to the aqueous solution of a sugar. The biological sample to be suspended can be prepared from a sample obtained from a living body as a raw material via various pretreatments. For example, when cancer cells in human blood are used as the biological sample, it is possible to exemplify the use of a sample obtained by pretreating the blood collected from human to remove platelet, erythrocyte, and so forth in accordance with a known technique, as the biological sample.

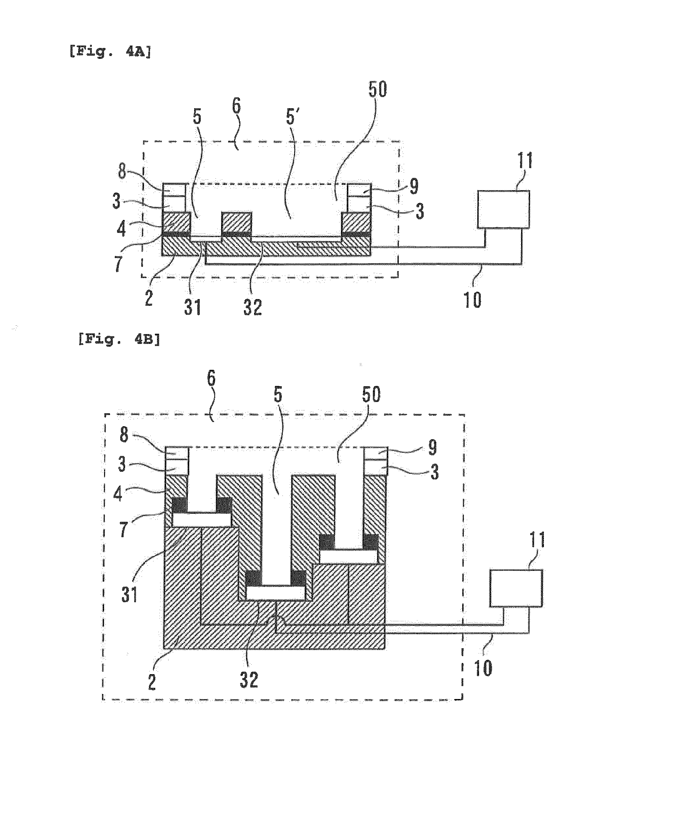

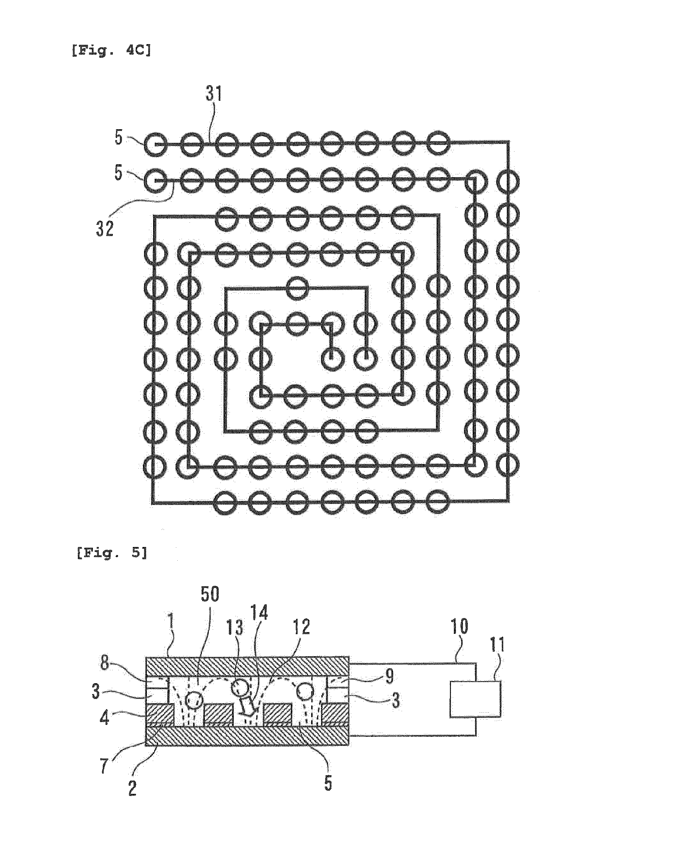

The apparatus exemplarily shown in Fig. 1 is configured such that the upper electrode substrate 1 is used as the upper lid to close the accommodating unit 50. By contrast, when the accommodating unit 50 is configured as a liquid reservoir without such an upper lid, it is possible to exemplify, for example, an apparatus configured as shown in Fig. 3. In the apparatus shown in Fig. 3, a pair of electrodes 31 and 32 is arranged on the lower electrode substrate 2, and the upper electrode substrate 1, which is included in the configuration shown in Fig. 1, is omitted. Fig. 4 is a figure showing a cross section of the apparatus shown in Fig. 3 along with B-B', wherein the pair of electrodes 31 and 32 is arranged on one sheet of the electrode substrate in a comb form.

The configuration of the immobilizing apparatus shown in Figs. 3 and 4 will be described in detail below. The pair of electrodes 31 and 32 has a so-called comb form (comb-shaped form). As shown in Fig. 3, each of the electrodes 31 and 32 has a plurality of small electrode portions (portions extending in a band form as shown in the drawing) that are arranged on one common flat surface positioned on the side of the holding portion (through-hole) 5 with respect to the spacer 3. The small electrode portions are connected to one another at their rear anchors, and further connected to the corresponding terminal of an AC power source 11. The small electrode portions of each of the electrodes are arranged alternately on one common flat surface. The holding portions (through-holes) 5, which are formed while penetrating through the insulator 4 and the light shielding member 7, are overlapped on the respective small electrode portions of the pair of electrodes 31 and 32 (see Fig. 4). Therefore, in Fig. 4, the electrode that corresponds to each of the holding portions (through-holes) 5 aligned adjacently in the horizontal direction is the electrode 31 or the electrode 32, and that is, the different electrodes 31 and 32 are adjacently aligned. In this configuration, the distance between the small electrode portion at the side of the electrode 31 and the small electrode portion at the side of the electrode 32 is kept to be a given distance between the electrodes. As described later on, the given distance between the electrodes refers to the distance which is sufficient to generate the dielectrophoresis of the biological sample as the target of the immobilization by the immobilizing apparatus, and the given distance between the electrodes can be appropriately determined while considering the characteristics of the biological sample or the like. In the case of the immobilizing apparatus configured as described above, the internal spaces of the accommodating unit 50 and the holding portions (through-holes) 5 correspond to the given dielectrophoretic space of the present invention. The respective small electrode portions of the electrodes 31 and 32 are arranged at the bottom portions of the holding portions (through-holes) 5, and these small electrode portions are arranged on one common flat surface. Therefore, when the AC voltage is applied between the electrodes, then the electric flux lines are allowed to pass between the holding portions (through-holes) 5 corresponding to the small electrode portions of the electrode 31 and the holding portions (through-holes) 5 corresponding to the small electrode portions of the electrode 32, and the dielectrophoretic force can be allowed to act on the biological sample intervening therebetween.

In the immobilizing apparatus having such a configuration of the electrodes, the distance through which the electric flux lines travel in the biological sample suspension can be shortened as compared with the immobilizing apparatus shown in Figs. 1 and 2, for the following reason. That is, in the case of the immobilizing apparatus shown in Fig. 1 or the like, the electric flux line passes between the pair of electrodes 1 and 2 separated from each other by the distance corresponding to the thickness of the spacer 3 or the like. By contrast, in the case of the immobilizing apparatus shown in Fig. 3 or the like, the electric flux line passes between the electrodes while basically excluding the thickness of the spacer 3. Further, the thicknesses of the insulator 4 and the light shielding member 7 are extremely smaller than the thickness of the spacer 3. By shortening the distance of the electric flux line passing between the electrodes as described above, it is possible to allow the dielectrophoretic force to effectively act on the biological sample contained in the suspension even in a state where the AC voltage applied between the electrodes is lowered. As a result, it is possible to appropriately ensure the miniaturization of the AC power source 11 or the maintenance of the insulation performance in the electrode configuration of the immobilizing apparatus.

Further, as shown in Fig. 3 or the like, the pair of electrodes 31 and 32 is arranged on one side with respect to the space for accommodating the suspension containing the biological sample, and thus the configuration of the immobilizing apparatus at the opposite side (upward side of the immobilizing apparatus as shown in Fig. 3 or the like) can be designed more freely. Accordingly, as shown in Fig. 3 or the like, it is possible to adopt the configuration in which the upper lid is not required for the immobilizing apparatus. As a result, the AC voltage is applied to the electrodes to allow the dielectrophoretic force to act on the biological sample, and thereby the biological sample is immobilized to the holding portion (through-hole) of the holding unit, while any arbitrary individual of the immobilized biological sample can be easily collected by using a micropipette or the like, or a given required solvent or the like is easily supplied for the biological sample after the immobilization. Also, when the biological sample is observed after the immobilization, due to the absence of a structure such as the lid, the signal (light emission or the like) to be observed, which is emitted from the biological sample, can be properly obtained in a state where attenuation of the signal is not caused by the lid or the like. On the other hand, it is also useful to provide the upper lid in order to obtain such an effect that the water in the suspension containing the biological sample introduced into the accommodating unit 50 is prevented from evaporation in the immobilizing apparatus shown in Fig. 3 or the like, or that the suspension containing the biological sample is stably supplied to the immobilizing apparatus in the immobilizing apparatus of the embodiment as shown in Fig. 3.

In the embodiment shown in Figs. 3 and 4, the plurality of holding portions (through-holes) are formed in the insulator 4 or the like. However, in principle, as shown in Fig. 4A, an embodiment in which one holding portion (through-hole) 5 is formed in the insulator 4 or the like is also within the scope of the biological sample immobilizing apparatus according to the present invention. That is, in this embodiment, one holding portion (through-hole) 5 is formed, and a diameter-expanded recess 5' is formed adjacently thereto. The diameter-expanded recess 5' has an opening diameter which is larger (for example, about several ten times) than that of the holding portion (through-hole) 5. One electrode 31 of the pair of electrodes is arranged on the bottom portion of the holding portion (through-hole) 5, and the other electrode 32 is arranged on the bottom portion of the diameter-expanded recess 5'. Further, the holding portion (through-hole) 5 and the diameter-expanded recess 5' are connected to the accommodating unit 50 on the upper opening side thereof.
In the case of such a configuration, the suspension exists in the internal space of the holding portion (through-hole) 5, the internal space of the diameter-expanded recess 5', and the accommodating unit 50, and hence, these spaces correspond to the given dielectrophoretic space according to the present invention. The electric flux line is generated between the electrodes when the voltage is applied between the pair of electrodes 31 and 32 in a state where the suspension containing the biological sample is put in the given dielectrophoretic space. However, on the side of the diameter-expanded recess 5', the density of the electric flux lines concentrated thereon is lowered, because the opening diameter is relatively large. By contrast, the electric flux lines are concentrated on the opening as described above on the side of the holding portion (through-hole) 5. Therefore, the effect to attract the biological sample contained in the suspension by means of the dielectrophoretic force can be ignored on the side of the diameter-expanded recess 5'. Therefore, the embodiment shown in Fig. 4A corresponds to such a mode that substantially only one holding portion (through-hole) 5 for holding the biological sample is formed. The electrode 32 is arranged together with the electrode 31 on the flat surface forming the internal space of the holding portion (through-hole) 5 while providing a given distance between the electrodes with respect to the electrode 31.
Therefore, similarly to the embodiment shown in Fig. 4, the biological sample can be immobilized to the one holding portion (through-hole) 5, and hence, it is possible to decrease the AC voltage applied between the electrodes as small as possible. Details of the effect to hold the biological sample will be described later on again.

In another embodiment in which one holding portion (through-hole) is provided, the biological sample suspension can be put outside the holding portion (through-hole) 5 by utilizing the surface tension on the surface of the insulator 4 or the like and then the voltage can be applied for the dielectrophoresis in a state where the biological sample suspension is present between the electrodes.

Further, an embodiment shown in Fig. 4B can be exemplified as another embodiment in which the AC voltage applied between the electrodes is decreased when the biological sample is immobilized. In the embodiment shown in Fig. 4B, similarly to the embodiment shown in Fig. 4, a plurality of holding portions (through-holes) 5 are provided and the respective upper openings of the holding portions (through-holes) 5 are connected to the accommodating unit 50. In this context, the embodiment shown in Fig. 4B and the embodiment shown in Fig. 4 are coincident with each other in that the pair of electrodes 31 and 32 are arranged on the side of a common installation position with respect to the holding portions (through-holes) 5, i.e., the pair of electrodes are arranged in the state of not interposing the accommodating unit 50 or the like in which the suspension is present. However, the embodiment shown in Fig. 4B differs in that the heights of the electrodes on the substrate 2 are not constant, in other words, in that the electrodes are arranged in a stepped form on the substrate 2. As a result, the depths of the holding portions (through-holes) 5 positioned on the respective electrodes differ depending on the places.

Also, in the case of the immobilizing apparatus configured as described above, the pair of electrodes are arranged in the state of not interposing the accommodating unit 50 or the like as described above, and hence, it is possible to decrease the application voltage for generating the dielectrophoretic force as small as possible. Further, the depths of the holding portions (through-holes) 5 provided on the electrodes are changed depending on the places, the diameters of the openings of the holding portions (through-holes) 5 are also appropriately changed if necessary, and thereby the different biological samples can be simultaneously immobilized to the holding portions (through-holes) 5 suitable for the respective samples. It is considered that the embodiment shown in Fig. 4B is useful, for example, when the suspension contains different biological samples, or when cells and aggregates thereof each are simultaneously immobilized.

Further, an immobilizing apparatus having a relative positional relationship between a pair of electrodes 31 and 32 and holding portions (through-holes) 5 shown in Fig. 4C is exemplified as another embodiment of the configuration in which no lid is provided as described above. Fig. 4C is a schematic view overlappedly showing the pair of electrodes 31 and 32 and the holding portions (through-holes) 5 formed in the insulator 4 or the like. In this embodiment, the pair of electrodes 31 and 32 each having a continuous line form (which can also be referred to as "band form" when the width of the electrode is taken into consideration) are arranged on a common flat surface, and the distance between the lines of the both electrodes is kept to be a given distance between the electrodes. As described later on, the given distance between the electrodes refers to the distance which is sufficient to generate the dielectrophoresis of the biological sample as the target of the immobilization by the immobilizing apparatus, and the given distance between the electrodes can be appropriately determined while considering the characteristics of the biological sample or the like. The holding portions (through-holes) 5 are arranged in an overlapped manner on the pair of electrodes 31 and 32 arranged as described above, and thereby the immobilization of the biological sample in such an embodiment that the distance that the electric flux line generated between the electrodes 31 and 32 in the biological sample suspension passes is shortened as much as possible can be realized in the holding portions (through-holes) 5 provided on the respective electrodes, as described with reference to Fig. 3 or the like. Further, similarly, the configuration shown in Fig. 4C is such an embodiment that the pair of electrodes are arranged on one side with respect to the space for accommodating the suspension containing the biological sample, and hence, the upper portion of the immobilizing apparatus can be configured to be opened, so that such a state that the immobilized biological sample can be easily accessed is provided.

Although Fig. 4C shows the configuration of the pair of electrodes each having a continuous polygonal line form, in place thereof, it is also allowable to adopt a configuration of a pair of electrodes each having a continuous curved line form. For example, each of the electrodes can also be formed in a spiral form. Also in this case, the distance between the pair of electrodes is the given distance between the electrodes.

In the above-described immobilizing apparatuses shown in Figs. 1 to 4C, a part of the accommodating unit 50 is composed of the insulator material so that the electric flux lines are concentrated on an arbitrary holding portion (through-hole) when the AC voltage is applied to the electrodes, and thereby the biological sample is moved and immobilize to the holding portion. Although it is preferable that the holding portions (through-holes) are arranged at equal intervals in the longitudinal and lateral directions, other than the above, for example, the holding portions (through-holes) can be arranged on a straight line at equal intervals only in the longitudinal direction or the latitudinal direction. This is because, when the holding portions (through-holes) are arranged as described above, then the electric field generated by the AC voltage applied between the electrodes is generated almost equivalently for all of the holding portions (through-holes), and the effect that the uniform operation is realized in the apparatus of the present invention is achieved.

The whole of the holding unit is composed of an insulator material, or at least a part of the holding unit is composed of an insulator. It is preferable that the insulator has the affinity for the biological sample, because the biological sample is attracted to and immobilized to the holding portion (through-hole) provided therein. Specifically, it is preferable to use a hydrophilic insulator when the biological sample is hydrophilic, while it is preferable to use a hydrophobic insulator when the biological sample is hydrophobic. The criterion for the affinity is generally represented by the contact angle formed between the liquid droplet and the surface of the insulator when a liquid having the affinity approximate to that of the biological sample is added dropwise to the surface of the insulator (the smaller the contact angle is, the higher the affinity between the liquid and the surface of the insulator is, while the larger the contact angle is, the lower the affinity between the liquid and the surface of the insulator is). Examples of the insulator having a relatively high hydrophilicity can include glass and titanium oxide, and examples of the insulator having a relatively high hydrophobicity include resins such as polystyrene, polyimide, Teflon (registered trademark). It is preferable to select and use these materials depending on the hydrophilicity and the hydrophobicity of the biological sample to be handled. Even when an insulator that intrinsically has the low affinity for the biological sample needs to be used, it is possible to enhance the affinity for the biological sample by reforming the surface of the insulator. As for the method for making the hydrophobic insulator such as the resin to be hydrophilic, it is appropriate to use known methods such as the plasma treatment, the chemical modification, and the modification based on the physical adsorption of protein or the like, methods in which these methods are arbitrarily combined, and so forth. As for the method for making the hydrophilic insulator such as the glass to be hydrophobic, it is appropriate to use a method based on the chemical modification in which a silane coupling agent is bound to a hydrophilic insulator surface.

In order to provide the through-holes which serve as the holding portions for the holding unit, it is possible to utilize various methods depending on the type of the insulator. For example, in order to provide the holding portions (through-holes) in the resin, it is possible to use known methods such as a method in which the laser is radiated and a method in which the resin is molded by using a mold having pins for providing the holding portions (through-holes). Also, when a light curing resin (photocuring resin) or the like is used, the holding portions (through-holes) can be provided by means of the general photolithography (exposure) and the etching (development) by using a photomask for exposure drawn with a pattern corresponding to the holding portions (through-holes).

The whole of the holding unit can also be composed of an insulator material. For example, as shown in Fig. 1, the member 4, which is a part of the holding unit, can be composed of an insulator material, and the light shielding member 7 can be composed of a non-insulator separately from the member 4. For example, it is possible to exemplify an embodiment in which the spacer 3 (or a frame in place thereof) is formed of a material which can be easily processed, the insulator 4 made of resin and the light shielding member 7 each provided with the holding portions (through-holes) are bonded to the frame or the spacer so that the suspension is prevented from being leaked, and thereby the holding portions of the holding unit and the accommodating unit 50 are communicated with each other. When the insulator 4 provided with the holding portions (through-holes) is a light shielding member or a member subjected to the light shielding treatment, it is possible to omit the light shielding member 7. The light shielding member is not particularly limited as long as it can shield the light (electromagnetic wave) intended to be detected. For example, a metal thin film can be exemplified as the light shielding member for the light having a wavelength of 380 nm to 780 nm as the visible light region. Considering the close contact performance with respect to the electrode substrate as the base material, a Cr metal thin film (film thickness: 100 nm) can be exemplified as a preferred metal thin film. When the light shielding member is installed between the insulator and the lower electrode substrate as described above to reduce the light noise around the holding portion (through-hole), it is possible, for example, to detect information such as the faint light emitted from the biological sample in the holding portion (through-hole) at a higher sensitivity.

The apparatus shown in Fig. 1 will be further explained. The spacer 3, which constitutes the accommodating unit 50, is provided to ensure the space for holding the suspension of the biological sample. The spacer 3 can be composed of an insulator such as glass, ceramic, and resin, as a material. The spacer 3 can also be composed of a conductor including such as metals as long as it provides a configuration in which the electric conduction is not provided between the upper electrode substrate 1 and the lower electrode substrate 2. In the example shown in Fig. 1, the spacer is provided with an introducing flow passage, an introducing port 8 communicated with the flow passage, a discharge flow passage for discharging the suspension, and a discharge port 9 communicated with the flow passage so that the supply and the discharge of the biological sample suspension to be fed to the apparatus can be quickly carried out. The size (dimensions) and the shape of the spacer 3 and the inner space and the thickness of the spacer can be determined in relation to the amount of the suspension accommodated in the accommodating unit 50.
They are not particularly limited, and in general, it is sufficient that such a volume that the biological sample suspension is introduced in an amount of several µL to several mL is provided. For example, when the size of the spacer approximately has length 40 mm x breadth 40 mm, then the inner space of the spacer can approximately have length 20 mm x breadth 20 mm, and the thickness of the spacer can be approximately 0.5 to 2.0 mm. The material of the electrode arranged on the electrode substrate is not particularly limited as long as a conductive and chemically-stable member is used. It is possible to use metals such as platinum, gold, copper, alloys such as stainless steel, and transparent conductive materials such as ITO (Indium Tin Oxide), and the like. In particular, when the electrode substrate is a transparent glass or the like for the purpose of monitoring the visual information such as the light obtained from the biological sample in the holding portion (through-hole) of the holding unit when the biological sample suspension is fed to the apparatus of the present invention to perform the analysis, then the ITO electrode is an especially preferred electrode in view of the transparency thereof, the film formation performance thereof, or the like.

Fig. 2 is a schematic view showing a sectional view of the apparatus shown in Fig. 1 along with A-A'. The spacer 3 constituting the accommodating unit 50, the lower electrode substrate 2, the insulator 4 and the light shielding member 7 constituting the holding unit, and the upper electrode substrate 1 are laminated. Examples of the laminating means can include a method in which a quick curing type adhesive agent is allowed to flow into a mold for the spacer to simultaneously perform the formation and the lamination of the spacer, a method in which the respective members are laminated by using an adhesive agent, a method in which the fusion is performed by heating in a pressurized state, and a method in which a resin having the surface stickiness such as PDMS (polydimethylsiloxane) or silicon sheet is used as spacers to manufacture these components, followed by being stuck and laminated under the pressure. An AC power source 11 is connected to the pair of electrodes of the apparatus via conductive lines 10. The AC power source 11 is not particularly limited as long as it can apply, between the electrodes, the AC voltage sufficient to generate the electric field for moving and immobilizing the biological sample to the holding portion (through-hole).
Specifically, for example, it is possible to exemplify a power source capable of applying the AC voltage having a waveform such as a sine wave, a rectangular wave, a triangular wave, or a trapezoidal wave at a peak voltage of about 1 V to 20 V and a frequency of about 100 kHz to 3 MHz. In particular, it is especially preferable to apply, between the electrodes, the AC voltage having such a waveform that the biological sample can be moved and only one individual of the biological sample can be immobilized to one holding portion (through-hole). As the AC voltage having such a waveform, it is preferable to use the rectangular wave. This is because, the rectangular wave instantaneously arrives at the preset peak voltage as compared with any case in which the waveform is the sine wave, the triangular wave, or the trapezoidal wave, and hence, the biological sample can be quickly moved toward the holding portion (through-hole) and it is possible to lower the probability that two or more individuals of the biological sample overlappedly enter the holding portion (through-hole) (it is possible to increase the probability that only one individual of the biological sample is immobilized to one holding portion (through-hole)). The biological sample can be regarded as a capacitor in an electrical viewpoint. During the period in which the peak voltage of the rectangular wave is not changed, the current hardly flows in the biological sample immobilized to the holding portion (through-hole), thereby the electric flux lines are hardly generated, and as a result, the dielectrophoretic force is hardly generated in the holding portion (through-hole) to which the biological sample is immobilized. Therefore, when the biological sample is once immobilized to the holding portion (through-hole), the probability that another biological sample is immobilized to the same holding portion (through-hole) is lowered. In place thereof, the biological sample is successively immobilized to the holding portion in which the electric flux line is generated and the dielectrophoretic force is generated (empty holding portion (through-hole) to which no biological sample is immobilized). In the apparatus of the present invention, it is preferable to adopt a power source which generates the AC voltage having no DC component, for the following reason. This is because, if the AC voltage having a DC component is applied, then the biological sample is moved while receiving the force biased to a specific direction due to the electrostatic force generated by the DC component, and the biological sample is hardly immobilized to the holding portion (through-hole) by means of the dielectrophoretic force. Also, if the AC voltage having a DC component is applied, then the ion contained in the suspension containing the biological sample causes the electric reaction on the electrode surface to generate the heat, thereby the biological sample causes the thermal motion on account thereof, and hence, it is impossible to control the motion by means of the dielectrophoretic force and it is difficult to move and immobilize the biological sample to the holding portion (through-hole).

In the apparatus of the present invention, the waveform of the applied AC voltage is preferably rectangular so that only one individual of the biological sample can be immobilized to one holding portion (through-hole). In order to achieve such an object, it is preferable that the arrangement, the size (dimensions), and the shape of the holding portion (through-hole) are set to be an arrangement, a size (dimensions), and a shape which are suitable for immobilizing only one individual of the biological sample to one holding portion (through-hole). For example, as for the arrangement, it is preferable that the holding portions (through-holes) are arranged in an array form in the holding unit. However, if the distance between the adjacent holding portions (through-holes) is too narrow, any favorable influence is not attained in the analysis of the biological sample, such as the following: the probability that a plurality of individuals of the biological sample is immobilized to one holding portion (through-hole) is increased; or the biological sample suspensions in the adjacent holding portions (through-holes) are mixed with each other. By contrast, if the distance between the adjacent holding portions (through-holes) is wide, then the biological sample remains at the position between the holding portion (through-hole) and the holding portion (through-hole), and hence, the probability that holding portion(s) (through-hole(s)) incapable of immobilizing the biological sample occur is increased.

In order to avoid the above-described problem, it is possible to exemplify that the distance between the adjacent holding portions (through-holes) is within a range between not less than three times and not more than twenty times the particle size of the biological sample to be immobilized. Further, from the viewpoint that it is sufficient that the holding portions (through-holes) are not in contact with each other in order that the dielectrophoretic force is allowed to act on the biological sample and thereby the biological sample is immobilized to the holding portion (through-hole), the distance is preferably not less than 1 µm to not more than 5000 µm, more preferably not less than 5 µm to not more than 2500 µm, and still more preferably not less than 10 µm to not more than 500 µm. In the next place, the size (dimensions), i.e. the diameter and the depth each, of the holding portion (through-hole) is preferably within a range between not less than twice and not more than five times the diameter of the biological sample. The size such as these can also be provided from the viewpoint that the dielectrophoretic force is allowed to act on the biological sample and thereby the biological sample can enter the holding portion (through-hole) so as to be held. For example, when the biological sample as the immobilization objective is a virus, a microorganism, a cell, or a tissue slice, the size of the holding portion can be set to be not less than 1 µm to not more than 1000 µm. In particular, when the biological sample as the immobilization objective is a cell or an aggregate thereof, it is preferable that the size of the holding portion is not less than 5 µm to not more than 500 µm. When the biological sample as the immobilization objective is a cancer cell or an aggregate thereof (composed of about 2 to 3 cells), it is preferable that the size of the holding portion is not less than 10 µm to not more than 100 µm. In this way, the electrostatic force is generated between the biological sample and the electrode surface on the bottom surface of the holding portion (through-hole), so that the biological sample is reliably immobilized to the holding portion (through-hole) and it is possible to secure the reaction space for performing the analysis.

The analysis method of the present invention is characterized in that the biological sample is immobilized to the holding portion (through-hole) of the holding unit, and then a specific gene of the biological sample is detected. The analysis method of the present invention will be explained below with reference to Figs. 5 to 8.

At first, as shown in Fig. 5, when the biological sample suspension is fed from the introducing port 8 of the spacer constituting the accommodating unit 50 and the AC voltage having the above-described waveform is applied, then the electric flux lines 12 are concentrated on the holding portion (through-hole 5 penetrating in the vertical direction) positioned just above the electrode, the dielectrophoretic force is allowed to act on the biological sample 13, and thereby the biological sample is moved along the electric flux line. As a result, one individual of the biological sample is immobilized to one holding portion (through-hole 5). The principle of the dielectrophoretic force will be explained below with reference to Fig. 5. The polarization arises in the dielectric particle of the biological sample 13 (a cell or the like) in the solution placed within the AC voltage, i.e. within the AC electric field, and the positive and negative electric charges are induced. In this situation, as shown in Fig. 5, when an uneven and ununiform electric field, i.e. the electric flux lines 12, is applied to the holding portion (through-hole) provided in the insulator arranged on the lower electrode substrate 2, the biological sample 13 is attracted to the direction in which the electric field is concentrated (direction in which the electric flux lines are dense), i.e. to the direction of the holding portion (through-hole). This is the dielectrophoretic force 14. In general, the dielectrophoretic force is proportional to the volume of the particle, the difference in the dielectric constant between the particle and the solution, and the square of the magnitude of the ununiform electric field. For example, when the AC electric field of 1 x 10⁵ to 5 x 10⁵ V/m having a frequency of 100 kHz to 3 MHz is applied as the electric field to the particle having a diameter of about 5 to 10 µm, then the dielectrophoretic force is allowed to act, so that the particle is attracted to the direction in which the electric field is concentrated. In this case, the biological sample is introduced to the holding portion (through-hole) mainly by the dielectrophoretic force, the gravity, and the electrostatic force from the electrodes. The number of the biological sample fed to the apparatus is not particularly limited. However, considering the effective use of the biological sample, it is preferable that the number is approximately equivalent to the number of the holding portions (through-holes) provided for the holding unit.

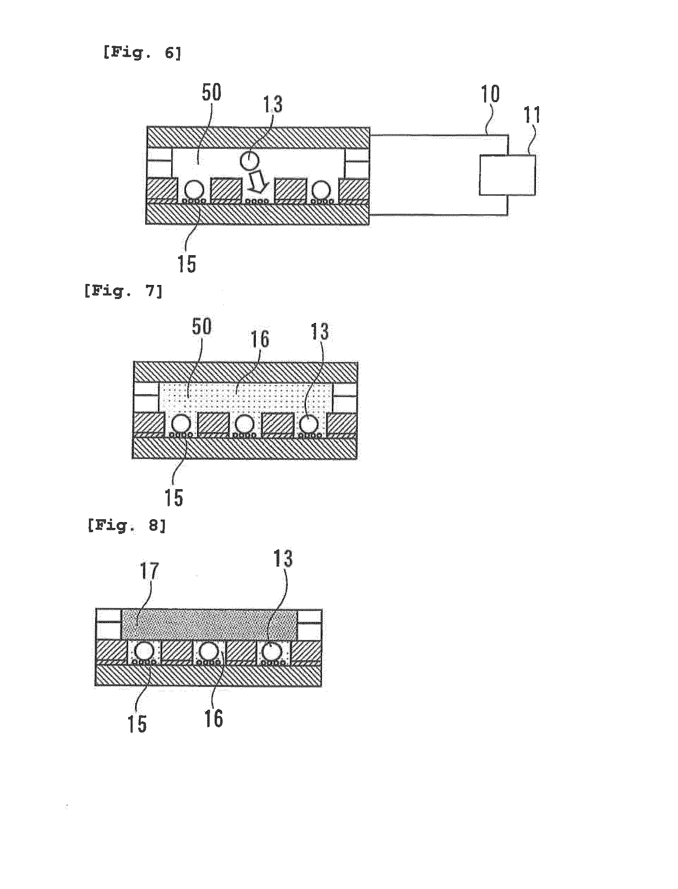

Subsequently, as shown in Fig. 6, the biological sample is bound to the inside of the holding portion (through-hole) modified with a substance 15 which binds to the biological sample, while applying the AC voltage. The substance which binds to the biological sample is not particularly limited as long as the substance specifically binds to the biological sample. Examples of the substance can include, for example, a molecule which recognizes a substance specifically present on the surface of the biological sample (ligand-receptor, sugar chain-lectin, antigen-antibody), and a Biocompatible Anchor for Membrane (BAM) having an aliphatic oleyl group, which binds to the lipid bilayer of the cell. Considering the binding to the biological sample in a relatively short period of time, poly-L-lysine, which electrostatically binds to the surface of the biological sample, can be exemplified as a preferred substance.

The method for modifying the holding portion (through-hole) with the substance which binds to the biological sample is not particularly limited as long as the biological sample can bind to the holding portion. For example, the inside of the holding portion (through-hole) can be specifically modified by utilizing the Au-thiol bond so that a substance having a thiol group, which binds to the biological sample, is reacted with an Au electrode on the bottom surface of the holding portion (through-hole). Further, after immobilizing the biological sample to the holding portion (through-hole), the holding portion (through-hole) and the biological sample can be bound to each other by supplying, to the accommodating unit 50, a solution containing the substance which binds to the biological sample. When the immobilization is performed by supplying the solution containing the substance which binds to the biological sample, it is preferable that the holding portion is washed after the binding reaction by using a solution for suspending the biological sample, such as an aqueous solution of a sugar such as mannitol, glucose, or sucrose, or an aqueous solution containing an electrolyte such as calcium chloride or magnesium chloride, or a protein such as BSA (bovine serum albumin) in the aqueous solution of a sugar so that component(s) that are not immobilized are removed. The biological sample immobilized to the holding portion (through-hole) via the substance which binds to the biological sample as described above is not disengaged from the holding portion (through-hole) even when the AC voltage is not continuously applied.

Subsequently, as shown in Fig. 7, a reagent solution for the analysis is injected into the accommodating unit 50. The reagent to be used for the analysis is not particularly limited as long as a specific gene can be detected. For example, it is possible to exemplify commercially available primers, probe, heat-resistant polymerase, usable for the real time PCR method and a fluorescent probe usable for the FISH method. In general, when the specific gene is detected, in many cases, a double strand DNA is thermally denatured to provide single strand DNAs by being heated at a high temperature, and then the detection is performed by reacting primers, a probe, and a heat-resistant polymerase with the single strand DNA, or by hybridizing a fluorescent probe complementary to the specific gene to the single strand DNA. Therefore, when the heating treatment is performed when the gene of the biological sample immobilized to the holding portion (through-hole) is analyzed, then the genetic material derived from the biological sample contained in the holding portion (through-hole) can be diffused to the outside of the holding portion (through-hole) due to the thermal convection, and the genetic material can contaminate the biological sample suspension in the adjacent holding portion (through-hole). In such a case, it is appropriate to add a temperature-responsive high molecular weight compound 16 into the reagent solution for the analysis. The temperature-responsive high molecular weight compound 16 is not particularly limited as long as it turns into a gel at a high temperature. Examples of the compound can include, for example, poly(vinyl methyl ether) (PVME), poly(methacrylic acid) (PMMA), polyethylene glycol (PEG), polypropylene glycol (PPG), methyl cellulose (MC), and hydroxypropyl cellulose (HPC). Considering that the biological sample suspension in the adjacent holding portion (through-hole) is not contaminated and the analysis of the biological sample is not inhibited, it is especially preferable to use poly(N-isopropyl acrylamide) (PNIPAAm) having the lower critical solution temperature (LCST) of about 32°C.

Further, as shown in Fig. 8, the holding portion (through-hole) to which the biological sample is bound is covered with a water-insoluble liquid 17, and thereby it is possible to avoid the contamination of the biological sample suspension in the adjacent holding portion (through-hole) and the evaporation of the biological sample suspension. The water-insoluble liquid is not particularly limited as long as it is not a water-soluble liquid. Examples of the water-insoluble liquid can include, for example, various oils and fluorine solvents. Considering that the biological sample suspension in the holding portion (through-hole) is not evaporated, the mixing with the biological sample suspension contained in the adjacent holding portion (through-hole) does not occur, and the analysis of the biological sample is not inhibited, a mineral oil can be exemplified as a preferred water-insoluble liquid. When the holding portion (through-hole) is covered with the water-insoluble liquid 17, then the holding portion (through-hole) can be covered by introducing the water-insoluble liquid into the accommodating unit 50 while installing the upper electrode substrate 1 to serve as the upper lid, or the holding portion (through-hole) can be covered with the water-insoluble liquid after removing the upper lid because the biological sample is immobilized to the holding portion (through-hole) via the substance which binds to the biological sample. Considering that the sample is collected after the analysis to further perform the detailed analysis, the latter method is more preferred.

Preferred examples of the method for detecting the specific gene can include the PCR method, the real time PCR method, the multiplex PCR method, and the FISH method. In particular, the real time PCR method, the FISH method, or the like, by which the specific gene can be amplified and detected by fluorescence while being immobilized to the holding portion (through-hole), or the fluorescent probe is allowed to bind to the specific gene to perform the detection so that the fluorescence coming from the holding portion (through-hole) to which the biological sample is immobilized can be directly detected, is preferred as the detecting method. Further, the apparatus of the present invention can also be adapted to the simultaneous measurement of multiple items. Therefore, as for the primers and the probes to be used for the analysis, a plurality of primers and probes of which nucleotide sequences are appropriately changed can be prepared to perform, for example, the specification of cancer stem cell in cancer cells and the identification and the distinction of mutant strain in the same species of bacterium or virus.

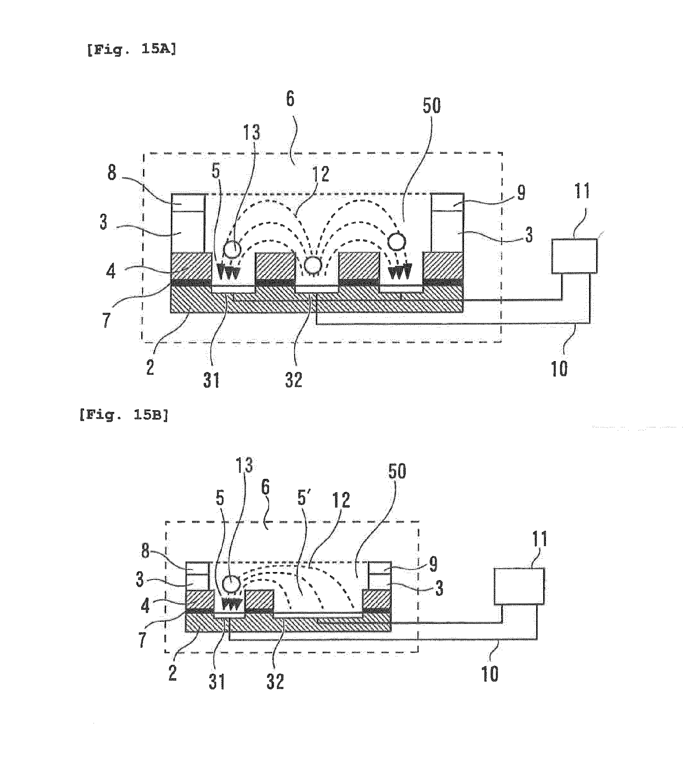

The above-described analysis method of the present invention based on Figs. 5 to 8 is the analysis method using the immobilizing apparatus shown in Figs. 1 and 2. The analysis method for the biological sample using the immobilizing apparatus shown in Figs. 3, 4, and 5 is also performed in intrinsically the same manner as shown in Fig. 15A. That is, when the AC voltage is applied to the pair of electrodes 31 and 32 formed in a comb form or a line form (band form), the electric flux lines 12 are concentrated between the mutually adjacent through-holes (holding portions) 5 positioned just above the electrodes. As a result, the biological sample receives the dielectrophoretic force, and one individual of the biological sample is immobilized to one holding portion (through-hole) 5. In the analysis method for the biological sample using the immobilizing apparatus shown in Fig. 4A, the electric flux lines 12 are generated as shown in Fig. 15B. That is, although the electric flux lines are generated between the electrode 31 which is provided at the bottom portion of one holding portion (through-hole) 5 and the electrode 32 which is provided at the bottom portion of the diameter-expanded recess 5', the diameter-expanded recess 5' has the wider electrode area, and hence, the density of the electric flux lines concentrated thereon is lower than the density of the electric flux lines concentrated on the holding portion (through-hole) 5. As a result, the immobilization of the biological sample is caused on the side of the holding portion (through-hole) 5, and the immobilization of the biological sample is not caused on the side of the diameter-expanded recess 5'. As a result, one individual of the biological sample is immobilized to one holding portion (through-hole) 5. Further, the above-described solvent or the like is appropriately supplied to the biological sample immobilized as shown in Figs. 15A and 15B, and thus the analysis of the biological sample is performed. In this case, no lid is provided at the upper portion of the immobilizing apparatus, and hence, such an advantage that, the solvent or the like can be supplied without requiring any labor to remove the lid, and further, the analysis operation is not inhibited by the lid during the analysis, can be pointed out.

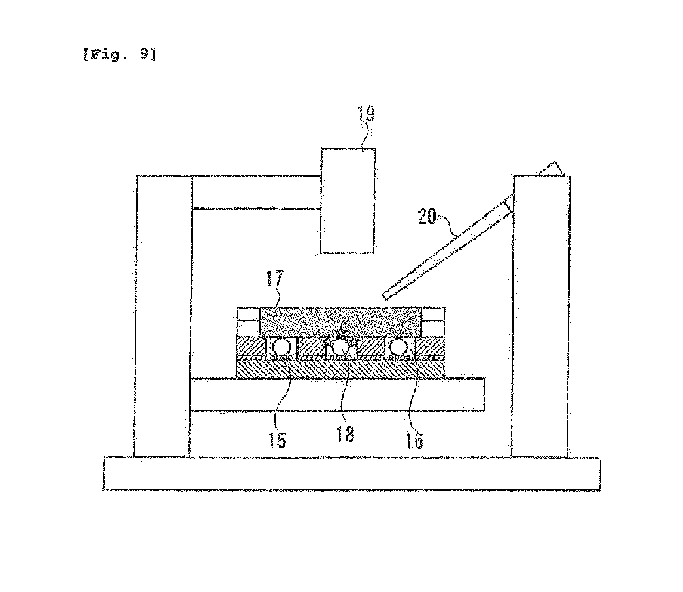

Fig. 9 shows an exemplary application of the apparatus of the present invention. For example, a suspension for which the presence of an abnormal cell 18 such as cancer is suspected is fed to the apparatus, and the cell is immobilized to the holding portions (through-holes). Also, a gene detection reagent for detecting a specific gene in the abnormal cell 18 as the objective of the detection is introduced, and the specific gene in the abnormal cell is amplified or a fluorescent probe is hybridized with the specific gene, so that the detection is performed.
Further, the abnormal cell such as cancer detected by a fluorescence microscope 19 can also be collected by using biological sample collecting means 20 (micropipette) to perform the analysis in further detail.

The micropipette has been explained above as the biological sample collecting means. However, the biological sample collecting means is not particularly limited as long as the biological sample can be collected. Other than the micropipette, it is possible to use a biological sample collecting means capable of precisely collecting (sampling) the biological sample by utilizing an electroosmotic flow. In the immobilizing apparatus shown in Figs. 3, 4, 4A, 4B, and 4C, no lid is originally provided at the upper portion of the immobilizing apparatus, and hence, it is easy to take out the biological sample by utilizing the micropipette or the like.

### EFFECT OF THE INVENTION

The apparatus of the present invention provides the following effects.
(1) The apparatus of the present invention makes it possible to quickly immobilize one individual of the biological sample to one holding portion (through-hole) provided in the holding unit. In particular, in the embodiment in which the holding unit is composed of the insulator provided with the plurality of through-holes arranged in the array form as the holding portions, a plurality of individuals of the biological sample can be quickly immobilized one by one to the holding portions (through-holes) arranged in the array form. Also, in the embodiment in which the pair of electrodes is arranged on the common flat surface, it is possible to contemplate the miniaturization of the power source required to apply the AC voltage.
(2) The apparatus of the present invention makes it possible to simultaneously analyze a plurality of individuals of the biological sample one by one while immobilizing the biological sample to the holding portions (through-holes). In particular, in the embodiment in which the pair of electrodes is arranged on the common flat surface, it is also possible to adopt such an embodiment that no structure to serve as the lid is installed to the upper portion of the apparatus, and hence, the biological sample can be analyzed more appropriately.
(3) The apparatus of the present invention makes it possible to collect the gene product derived from the biological sample immobilized to the holding portion (through-hole). In particular, in the embodiment in which the pair of electrodes is arranged on the common flat surface, it is also possible to adopt such an embodiment that no structure to serve as the lid is installed to the upper portion of the apparatus, and hence, the biological sample can be obtained more easily.

### BRIEF DESCRIPTION OF THE DRAWINGS

[Fig. 1] Fig. 1 shows a figure for illustrating an apparatus of the present invention.
[Fig. 2] Fig. 2 shows a sectional view of a main body 6 of the apparatus shown in Fig. 1 along with AA'.
[Fig. 3] Fig. 3 shows a figure for illustrating an apparatus having no upper lid of the present invention.
[Fig. 4] Fig. 4 shows a sectional view of a main body 6 of the apparatus shown in Fig. 3 along with BB'.
[Fig. 4A] Fig. 4A shows a sectional view illustrating a schematic configuration of another embodiment regarding the apparatus shown in Fig. 4.
[Fig. 4B] Fig. 4B shows a sectional view illustrating a schematic configuration of another embodiment regarding the apparatus shown in Fig. 4.
[Fig. 4C] Fig. 4C shows a relative positional relationship between a pair of electrodes and holding portions (through-holes) in another embodiment of the apparatus having no upper lid of the present invention.
[Fig. 5] Fig. 5 shows a figure for illustrating an analysis method using the apparatus of the present invention.
[Fig. 6] Fig. 6 shows a figure for illustrating the analysis method using the apparatus of the present invention.
[Fig. 7] Fig. 7 shows a figure for illustrating the analysis method using the apparatus of the present invention.
[Fig. 8] Fig. 8 shows a figure for illustrating the analysis method using the apparatus of the present invention.
[Fig. 9] Fig. 9 shows an example in which the apparatus of the present invention is applied as an apparatus for detecting an abnormal cell.
[Fig. 10] Fig. 10 shows schematic drawings to depict the steps of manufacturing a substrate in which a holding unit composed of a light shielding member and an insulator provided with holding portions (through-holes) and electrodes are integrated into one unit, by using the general photolithography and etching.
[Fig. 11] Fig. 11 shows a figure for illustrating the apparatus used in the present invention and Example 1.
[Fig. 12] Fig. 12 shows a sectional of a main body 6 of the apparatus shown in Fig. 11 along with CC'.
[Fig. 13] Fig. 13 shows an apparatus installed with the apparatus of the present invention and a micropipette as a biological sample collecting means for collecting a specific cell immobilized to a holding portion (through-hole) or a gene.
[Fig. 14A] Fig. 14A shows first drawings to depict the steps of manufacturing a substrate in which a holding unit composed of a light shielding member and an insulator provided with holding portions (through-holes) and electrodes are integrated into one unit, corresponding to the apparatus shown in Fig. 3.
[Fig. 14B] Fig. 14B shows second drawings to depict the steps of manufacturing the substrate in which the holding unit composed of the light shielding member and the insulator provided with the holding portions (through-holes) and the electrodes are integrated into one unit, corresponding to the apparatus shown in Fig. 3.
[Fig. 14C] Fig. 14C shows first drawings to depict the steps of manufacturing a substrate in which a holding unit composed of a light shielding member and an insulator provided with holding portions (through-holes) and electrodes are integrated into one unit, corresponding to the apparatus shown in Fig. 4B.
[Fig. 14D] Fig. 14D shows second drawings to depict the steps of manufacturing the substrate in which the holding unit composed of the light shielding member and the insulator provided with the holding portions (through-holes) and the electrodes are integrated into one unit, corresponding to the apparatus shown in Fig. 4B.
[Fig. 15A] Fig. 15A shows a figure for illustrating an analysis method using the apparatus of the present invention.
[Fig. 15B] Fig. 15B shows a figure for illustrating an analysis method using the apparatus of the present invention.

### MODES FOR CARRYING OUT THE INVENTION

The present invention will be explained in further detail below on the basis of Examples. However, the present invention is not limited to the Examples.

### Example 1

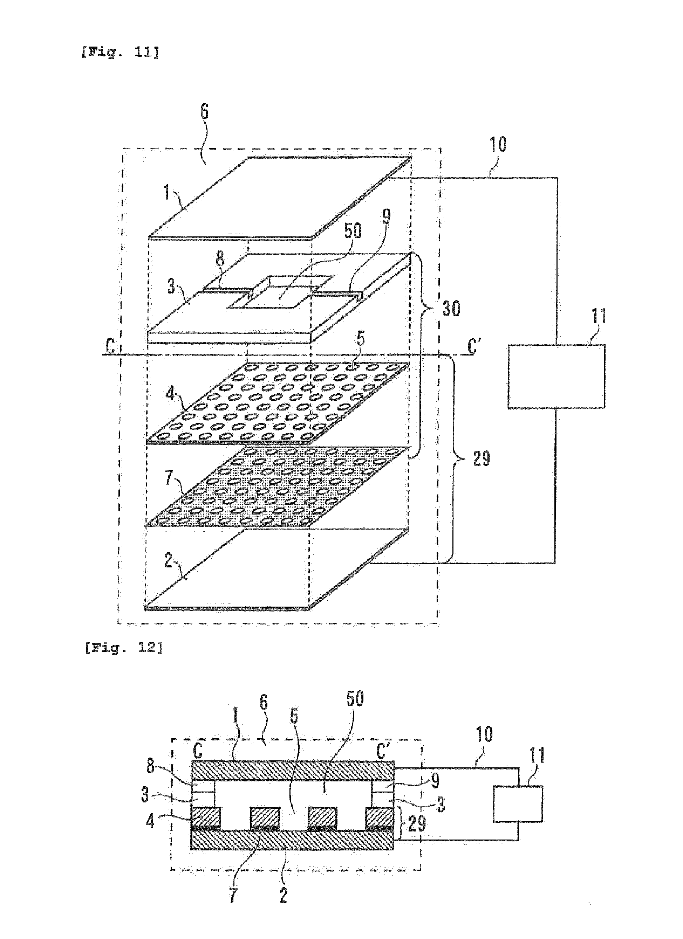

In Example 1, an apparatus was used, which had such a structure that a spacer 3 was arranged between an upper electrode substrate 1 and a lower electrode substrate 2, and a holding unit composed of a light shielding member 7 and an insulator 4 arranged with a plurality of circular holding portions (through-holes 5) in an array form was interposed by the spacer and the lower electrode as shown in Fig. 11.

A glass substrate having length 78 mm x breadth 56 mm x thickness 1 mm was used for the electrode substrate. The spacer 3 was prepared with Araldite (registered trademark) resin so that a space of length 20 mm x breadth 20 mm x thickness 1.5 mm was formed on the lower electrode substrate. The spacer was provided with an introducing port 8 and a discharge port 9 for introducing and discharging the suspension containing the biological sample.

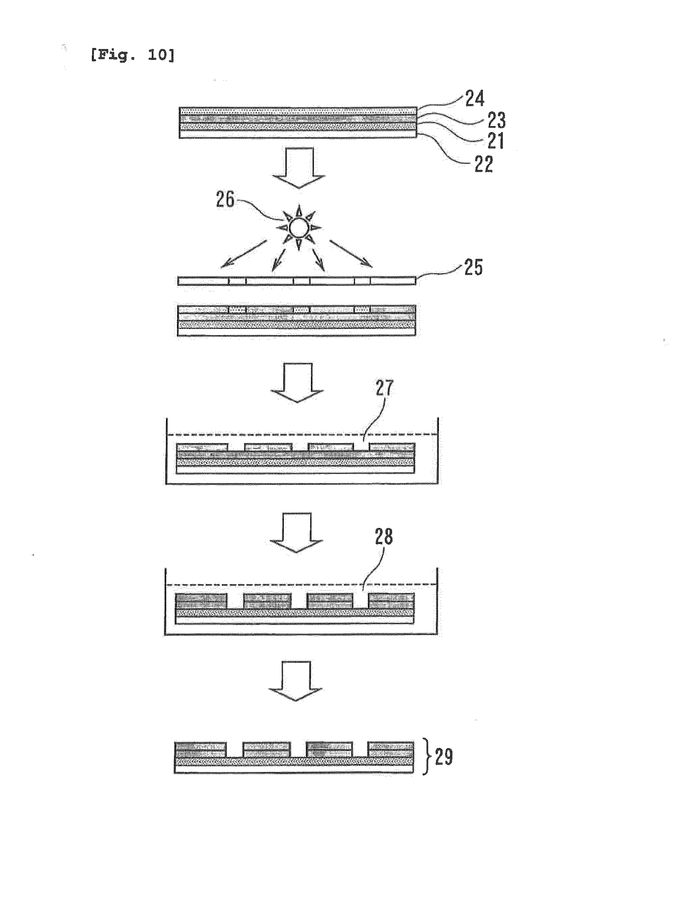

The insulator 4 provided with the plurality of holding portions (through-holes 5) was formed integrally with the lower electrode on the lower electrode substrate by means of a method based on the photolithography and the etching shown in Fig. 10. On the ITO film formation surface of a glass 22 on which ITO 21 had been formed as a film, Cr 23 having a film thickness of 100 nm was formed as a film by means of the sputtering. Subsequently, a resist 24 was applied onto the formed Cr so that the film thickness was 45 µm by using a spin coater. After performing natural drying for 1 minute, the prebaking (95°C, 15 minutes) was performed by using a hot plate. An epoxy-based negative type resist was used as the resist. Subsequently, by using a photomask 25 for exposure on which a pattern of micropores having diameters of φ34 µm and arranged in an array form composed of 150 pieces (length) x 150 pieces (breadth) with the longitudinal and latitudinal distances between the holding portion (through-hole) and the holding portion (through-hole) of 200 µm was depicted in an area of length 30 mm x breadth 30 mm, the resist was subjected to the exposure 26 by means of a UV exposure apparatus, followed by being developed with a developing solution 27. The exposure time and the developing time were adjusted so that the depth of the holding portion (through-hole) was 45 µm, which was equal to the film thickness of the resist. After the development, the exposed Cr film was exfoliated by means of 30% ceric ammonium nitrate solution 28 so that ITO was exposed at the bottom surface of the holding portion (through-hole). After that, the postbaking (150°C, 15 minutes) was performed by using a hot plate to cause the curing of the resist, and thereby to manufacture a lower electrode substrate integrated with the insulator provided with the through-holes.

The spacer 3 was manufactured as shown in Fig. 11 on the electrode substrate 29 manufactured as described above. Fig. 12 shows a sectional view of the analysis container (vessel) shown in Fig. 11 along with C-C'. As for the spacer, Araldite (registered trademark) adhesive agent of the quick curing type was poured into a mold of the spacer, and the respective parts were adhered in accordance with a method in which the formation of the spacer and the lamination were simultaneously performed. The suspension containing the biological sample was able to be accommodated into the accommodating unit without any leakage. The areal size cut out from the spacer was length 20 mm x breadth 20 mm, and hence the number of through-holes existing in the space was about 10,000. A power source 11 (signal generator) for applying the voltage between the electrodes was connected via conductive lines 10.

Mouse myeloma cells (particle size: about 10 µm) were used as the biological sample. The cells were suspended in a mannitol aqueous solution having a concentration of 300 mM to prepare a cell suspension so that the density was 1.25 x 10⁴ cells/mL.

Subsequently, 400 µL of the above-described cell suspension was injected in two parts from the introducing port of the spacer 3 by using a syringe (number of introduced cells: about 10,000 cells), and a rectangular wave AC voltage having a voltage of 20 Vpp and a frequency of 3 MHz was applied between the electrodes as the AC voltage by means of the signal generator. As a result, the cells were successfully immobilized one by one to the respective holding portions (through-holes) arranged in the array form within an extremely short period of time of about 2 to 3 seconds. The phrase "was/were successfully immobilized" means the case in which the cell has entered the holding portion (through-hole). The same definition was also used in Comparative Example described below. In this case, the biological sample immobilization rate, at which rate approximately one cell enters one holding portion (through-hole), was about 90%. The biological sample immobilization rate is defined by the value which is obtained by dividing the number of the holding portions (through-holes) in each of which one individual of the biological sample has entered by 225, when the biological sample is introduced and immobilized, while viewing 225 pieces of the holding portions (through-holes) composed of 15 pieces (length) x 15 pieces (breadth) in the field of a microscope. The same definition is also given in Examples and Comparative Example described later on.

400 µL of poly-L-lysine having a concentration of 2.5 x 10⁻⁴% was injected into the holding unit in which approximately one cell was immobilized to one holding portion (through-hole). After static placement for 3 minutes, a mannitol aqueous solution having a concentration of 300 mM was injected so as to wash poly-L-lysine in the accommodating unit. Thus, the cells were electrostatically bound to the inside of the holding portions (through-holes) successfully.

Subsequently, the β-action gene in the mouse myeloma cell was amplified. A solution having a composition shown in Table 1 was prepared and injected into the accommodating unit, then all of the holding portions (through-holes) were hermetically sealed with a mineral oil, and PCR was performed by using a thermal cycler. The condition of the heat cycle was such that preheat at 95°C for 3 minutes was performed, and then 40 cycles each consisting of a process at 95°C for 30 seconds and a process at 60°C for 40 second were performed.

**Table 1: Composition of Solution for PCR**

| Reagent | Concentration |
|---|---|
| Mouse ACTB (actin, beta) | |
| Endogeneous control | x 1 |
| dNTP | 200 µM |
| AmpliTaq Gold | 0.25 U/µL |
| GeneAmp PCR Buffer (MgCl₂) | x 1 |
| PNIPAAm solution | 1.5% |

The amplification of the β-action gene was confirmed by means of the TaqMan method, which is a method for performing the analysis based on the fluorescence. In the TaqMan method, an oligonucleotide in which the 5' end is modified with a fluorescent substance and the 3' end is modified with a quencher substance is utilized as the probe. In the case of this probe, the emission of the fluorescence is suppressed, because as well as the fluorescent substance, the quencher substance is present near the fluorescent substance. When the TaqMan probe hybridized with a gene strand intended to be detected is decomposed by the 5' to 3' exonuclease activity possessed by Taq DNA polymerase in the step of the elongation reaction of PCR, then the fluorescent dye is liberated from the probe, the suppression by the quencher is removed, and thereby the fluorescence is emitted. The fluorescence intensity was observed by means of a CCD camera with a fluorescence microscope (U-RFL-T/IX71 produced by Olympus Corporation). As a result, as compared with a fluorescence microscope image of the holding portion (through-hole) to which the cell was immobilized before performing PCR, the fluorescence intensity was increased in a fluorescence microscope image after performing PCR, and hence, the β-actin gene was successfully detected. By contrast, the increase in the fluorescence intensity according to the amplification of the β-action gene was unable to be detected in the holding portion (through-hole) to which no cell was immobilized.

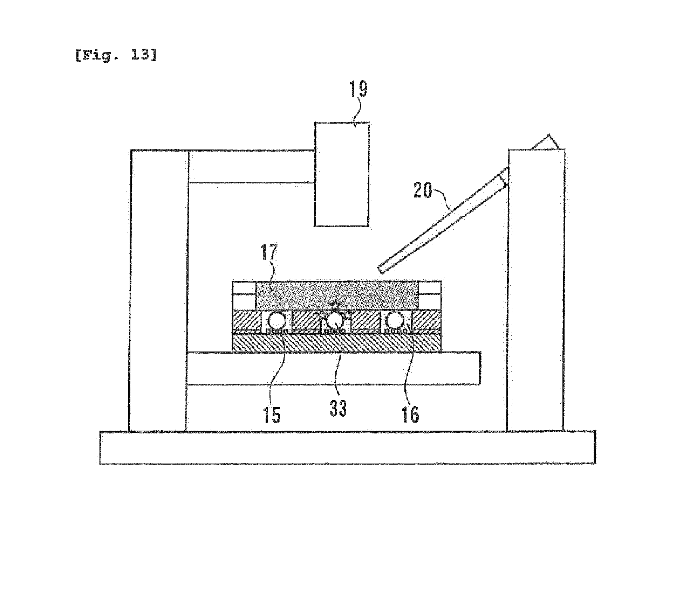

As shown in Fig. 13, a biological sample collecting means 20 was installed. A pipette capable of precisely sampling (collecting) the biological sample by utilizing the electroosmotic flow was used as the biological sample collecting means, and thereby a specific cell 33 immobilized to the holding portion (through-hole) and the β-action gene in the holding portion (through-hole) were successfully sampled (collected), while performing the observation with the microscope 19. The β-action gene was verified. The electrophoresis analysis was performed by using 3.0% agarose gel, and thus a single fragment of 115 bp indicating β-action was successfully confirmed from the holding portion (through-hole) from which the fluorescence was successfully detected. By contrast, the fragment was unable to be detected at all from the holding portion (through-hole) from which no fluorescence was detected. Cloning was performed into pMD20 vector (Takara) by using a PCR product obtained by reamplifying the β-action gene collected via the biological sample collecting means. The PCR product was ligated into pMD20 vector, and then ●BR>Y ligation reaction mixture was used to transform competent cells (Competent Cell, JM109, Takara). Clones containing pMD20 vector having the insertion fragment were selected on the basis of the presence of white colonies on Luria-Bertani (LB) plate containing 20 µg/mL X-GAL, 10 mM IPTG, and 50 µg/mL ampicillin. Seven colonies were picked up for each of the PCR samples, followed by being proliferated overnight in the LB medium. Subsequently, Quiagen QIAprep Miniprepkit (trade name) was used to isolate plasmid DNA. An obtained sequence was analyzed by using BLAST software to confirm that the sequence was completely coincident with that of the mouse β-action gene.

### Comparative Example

For the purpose of comparison, the following operation was performed by using the same apparatus as that used in Example 1. At first, 400 µL of the above-described cell suspension was injected in two parts from the introducing port of the spacer by using a syringe (number of cells: about 10,000 cells), and a rectangular wave AC voltage having a voltage of 20 Vpp and a frequency of 3 MHz was applied between the electrodes as the AC voltage by means of the signal generator. As a result, the cells were successfully immobilized one by one to the respective holding portions (through-holes) arranged in the array form within an extremely short period of time of about 2 to 3 seconds.

400 µL of poly-L-lysine having a concentration of 2.5 x 10⁻⁴% was injected into the accommodating unit in which approximately one cell was immobilized to one holding portion (through-hole). After static placement for 3 minutes, a mannitol aqueous solution having a concentration of 300 mM was injected so as to wash poly-L-lysine in the accommodating unit. Thus, the cells were electrostatically bound to the inside of the holding portions (through-holes) successfully.

The β**-**actin gene in the mouse myeloma cell was amplified. A solution having a composition shown in Table 2 was prepared and injected into the accommodating unit, then all of the holding portions (through-holes) were hermetically sealed with a mineral oil, and PCR was performed by using a thermal cycler. The condition of the heat cycle was such that preheat at 95°C for 3 minutes was performed, and then 40 cycles each consisting of a process at 95°C for 30 seconds and a process at 60°C for 40 second were performed.

**Table 2: Composition of Solution for PCR**

| Reagent | Concentration |
|---|---|
| Mouse ACTB (actin, beta) | |
| Endogeneous control | x 1 |
| dNTP | 200 µM |
| AmpliTaq Gold | 0.25 U/µL |
| GeneAmp PCR Buffer (MgCl₂) | x 1 |
| Sterilized water | - |

The amplification of the β-actin gene was confirmed by means of the TaqMan method, which is a method for performing the analysis based on the fluorescence. The fluorescence intensity was observed by means of a CCD camera with a microscope. As a result, as compared with a fluorescence microscope image of the holding portion (through-hole) to which the cell was immobilized before performing PCR, the increase in the fluorescence intensity was unable to be confirmed in a fluorescence microscope image after performing PCR. It was speculated that the gene and the fluorescent dye in the holding portion (through-hole) was diffused to the outside of the through-hole.

### Example 2

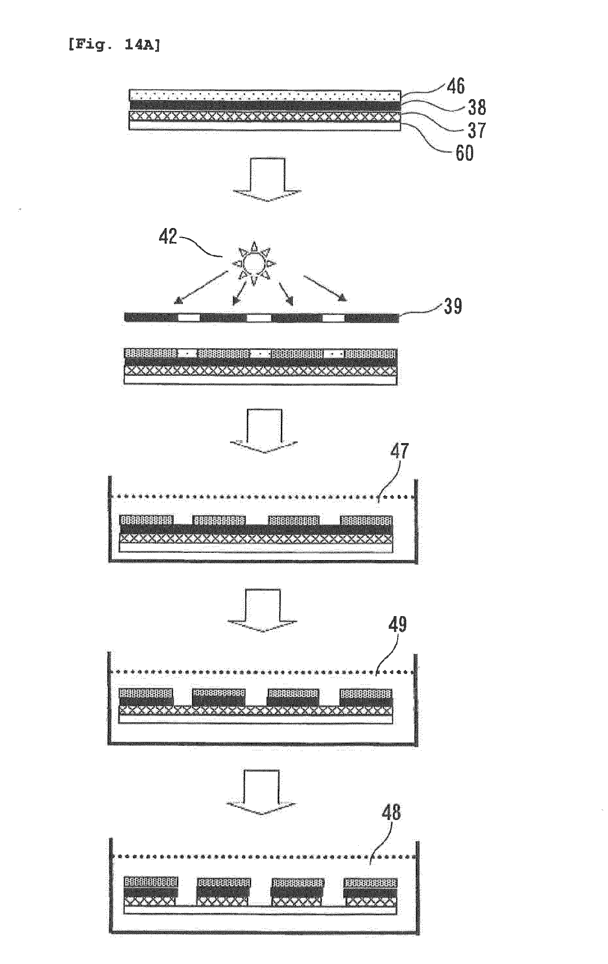

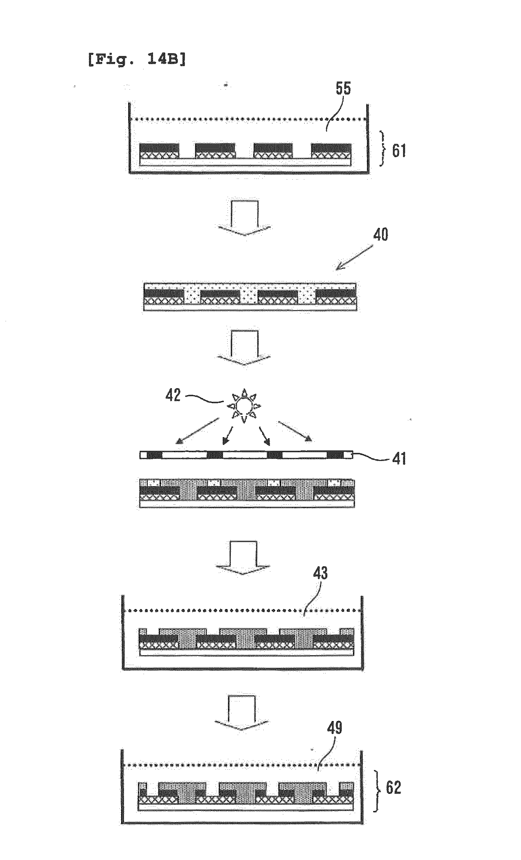

Next, in Example 2, an apparatus shown in Figs. 3 and 4 will be referred to, which has such a structure that a pair of electrodes 31 and 32 is provided on one side with respect to the suspension containing the biological sample, i.e., such a structure that a comb-shaped electrode pair is provided. In Example 2, a glass substrate having length 70 mm x breadth 40 mm x thickness 1 mm was used for the substrate on which the pair of electrodes was to be arranged. The spacer 3 was manufactured by cutting out a central portion of length 20 mm x breadth 20 mm from a silicon sheet having length 40 mm x breadth 40 mm x thickness 1.5 mm. An introducing port 8 and a discharge port 9 for introducing and discharging the suspension containing the biological sample were provided for the spacer 3. A holding unit having a plurality of holding portions (through-holes) 5 and the pair of electrodes 31 and 32 were formed integrally on the glass substrate in accordance with a method based on the photolithography and the etching shown in Figs. 14A and 14B.

As shown in Figs. 14A and 14B, ITO 37 having a film thickness of 100 nm was formed as a film by means of the sputtering on one surface of the glass substrate 60. Subsequently, Cr 38 having a film thickness of 100 nm was formed as a film on the formed ITO by means of the sputtering. Subsequently, a resist 46 was applied onto the formed Cr so that the film thickness was 1 µm by using a spin coater. After performing natural drying for 1 minute, the prebaking (105°C, 15 minutes) was performed by using a hot plate. A positive type resist was used as the resist.

Subsequently, by using a photomask 39 for exposure on which a comb-shaped electrode pattern in which band-shaped electrodes a each having a width of 10 µm and band-shaped electrodes b each having a width of 10µm were formed at intervals of 50 µm was depicted in an area of length 30 mm x breadth 30 mm, the resist was subjected to the exposure 42 by means of a UV exposure apparatus, followed by being developed with a developing solution 47. The exposure time and the developing time were adjusted so that the film thickness exfoliated by the development was 1 µm, which was equal to the film thickness of the resist. After the development, the exposed Cr film was exfoliated by means of 30% ceric ammonium nitrate solution 49 so that ITO 37 was exposed at the bottom surface of the through-hole. Subsequently, ITO etching solution (ITO-Etchant, Wako Pure Chemical Industries, Ltd.) 48 was used to exfoliate the exposed ITO film. Subsequently, as shown in Fig. 14B, the resist was exfoliated by means of a remover 55 to form the pair of comb-shaped electrodes 61 in which the Cr film was arranged on the ITO film.

A resist 40 was applied onto the substrate manufactured as described above by using a spin coater so that the film thickness was 5 µm. After performing natural drying for 1 minute, the prebaking (95°C, 3 minutes) was performed by using a hot plate. An epoxy-based negative type resist was used as the resist. Subsequently, by using a photomask 41 for exposure on which a pattern of micropores having diameters of φ8.5 µm and aligned in an array form composed of 600 pieces (length) x 600 pieces (breadth) at an interval of 50 µm was depicted in an area of length 30 mm x breadth 30 mm, the resist 40 was exposed by means of a UV exposure apparatus 42 in a state where the micropores were positionally adjusted on the comb-shaped electrodes, followed by being developed with a developing solution 43. The exposure time and the developing time were adjusted so that the depth of the hole was 5 µm, which was equal to the film thickness of the resist 40. After the development, the exposed Cr film was exfoliated by means of 30% ceric ammonium nitrate solution 49 so that ITO 37 was exposed at the bottom surface of the holding hole. After that, the postbaking (180°C, 30 minutes) was performed by using a hot plate to cause the curing of the resist, and thereby to manufacture a comb-shaped electrode substrate 62 integrated with the holding unit (stack of the insulator film and the light shielding film) formed with the plurality of holding holes.

The spacer 3 was stacked and adhered under pressure as shown in Figs. 3 and 4 on the holding unit on the comb-shaped electrode substrate manufactured as described above. The surface of the silicon sheet has the stickiness, and hence, the spacer 3 and the insulator 4 were successfully laminated by being adhered under pressure. The areal size of the accommodating unit of the spacer 3 is length 20 mm x breadth 20 mm, and hence the number of the holding portions (through-holes) 5 existing in the accommodating unit is about 160,000. A power source (signal generator) was connected to both of the pair of electrodes constituting the comb-shaped electrodes via conductive lines 10.

Mouse spleen cells (particle size: about 6 µm) were used as the biological sample. The calls were suspended in a mannitol aqueous solution having a concentration of 300 mM to prepare a cell suspension so that the density was 2.7 x 10⁵ cells/mL.

Subsequently, 600 µL of the above-described cell suspension was injected from the introducing port 8 of the spacer 3 by using a syringe (number of introduced cells: about 160,000 cells), and a rectangular wave AC voltage having a voltage of 20 Vpp and a frequency of 3 MHz was applied between the electrodes by means of the signal generator. As a result, the cells were successfully immobilized one by one to the respective holes of the plurality of holding holes formed in the array form within an extremely short period of time of about 2 to 3 seconds. Subsequently, 600 µL of poly-L-lysine having a concentration of 2.5 x 10⁻⁴% was injected into the accommodating unit. After static placement for 3 minutes, the application of the voltage was stopped. Subsequently, a phosphate buffer (pH 7.2) was injected so as to wash poly-L-lysine in the accommodating unit. Thus, the cells were electrostatically bound to the inside of the holding holes successfully.

Subsequently, B cell in the mouse spleen cell population was detected. The specific substance as the target for detecting B cell was CD19 molecule present on the surface of B cell. CD19 molecule is the B cell surface receptor, which is found on the cell through the entire differentiation of B cell line, in which B cell differentiates from the stage of the stem cell to finally into the plasma cell. Examples of B cell line can include pre-B cell, B cell (including naive B cell, antigen-stimulated B cell, memory B cell, plasma cell, and B lymphocyte), and follicular dendritic cell.

Subsequently, 600 µL of PE-labeled CD19 antibody (Miltenyi Biotec, Bergisch Gladbach, Germany) as a labeled substance was fed to the accommodating unit to label B cell via the antigen-antibody reaction (4°C, 10 minutes). After that, the washing was performed with a phosphate buffer, and the detection of B cell was carried out. The labeled B cell was observed by means of a CCD camera with a fluorescence microscope (U-RFL-T/IX71, Olympus Corporation, Japan). As a result, as compared with a fluorescence microscope image of the cell before the labeling, the fluorescence intensity only on the surface of B cell was increased after the labeling, and hence, B cell was successfully detected. Further, B cell was successfully collected by using the above-described micropipette.

### Example 3

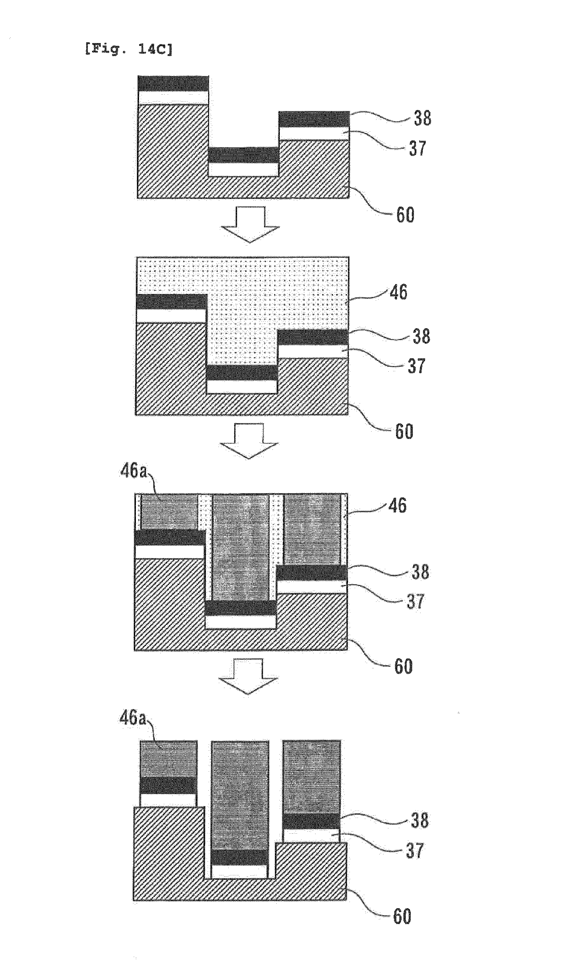

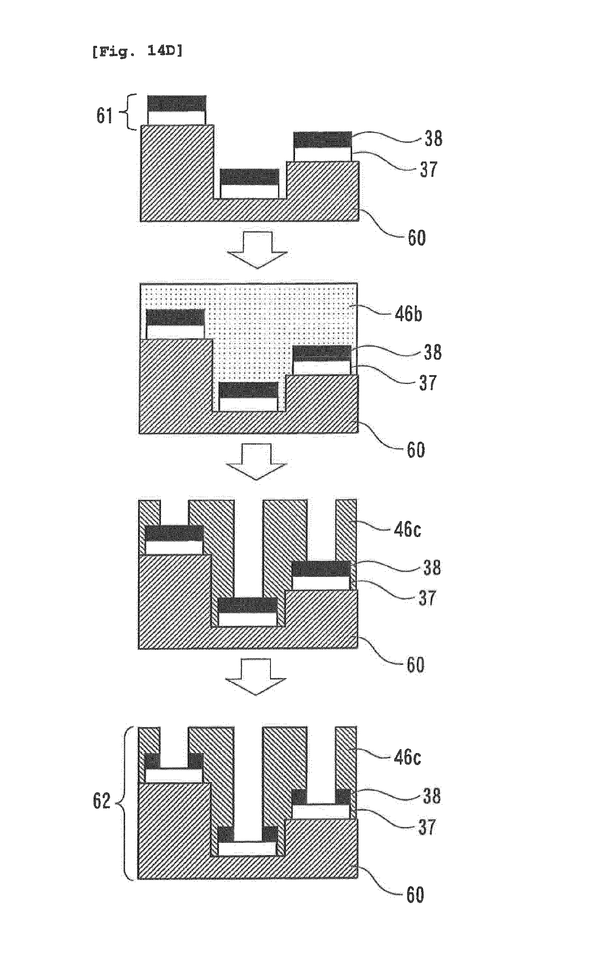

Next, in Example 3, an apparatus shown in Fig. 4B will be referred to, which has such a structure that a pair of electrodes 31 and 32 is provided on one side with respect to the suspension containing the biological sample, i.e., such a structure that a comb-shaped electrode pair is provided, wherein the electrodes are arranged in a stepped form on the substrate. In Example 3, in the same manner as in Example 2, a glass substrate 60 having length 70 mm x breadth 40 mm x thickness 1 mm was used for the substrate on which the pair of electrodes was to be arranged. However, the surface of the glass substrate 60 is processed in accordance with a technique or the like in which the etching rate is changed, and thereby stepped portions (or grooves) are formed on the surface. The stepped portions provide the stepped form in which the electrodes are to be arranged. The spacer 3 and the introducing port 8 and the discharge port 9 provided for the spacer 3 are similar to those of Example 2. In Example 3, a holding unit having a plurality of holding portions (through-holes) 5 and the pair of electrodes 31 and 32 are formed integrally on the glass substrate 60 having the stepped surface in accordance with a method based on the photolithography and the etching shown in Figs. 14C and 14D.

As shown in Figs. 14C and 14D, ITO 37 having a film thickness of 100 nm was formed as a film by means of the sputtering on one surface of the glass substrate 60 having the stepped surface. Subsequently, Cr 38 having a film thickness of 100 nm was formed as a film on the formed ITO by means of the sputtering. Subsequently, a resist 46 was applied onto the formed Cr so that the height of the resist was constant irrelevant to the stepped form of the glass substrate 60 by using a spin coater. After performing natural drying for 1 minute, the prebaking (105°C, 15 minutes) was performed by using a hot plate. A positive type resist was used as the resist.

Subsequently, the resist 46 was exposed by means of a UV exposure apparatus by using a photomask for exposure not shown in the figure so that a resist 46a having a constant width remained on the ITO layer of each of the stepped portions, followed by being developed with a developing solution. After the development, the exposed Cr film was exfoliated by means of 30% ceric ammonium nitrate solution, and further, ITO etching solution (ITO-Etchant, Wako Pure Chemical Industries, Ltd.) was used to exfoliate the exposed ITO film. Subsequently, as shown in Fig. 14D, the resist was exfoliated by means of a remover to form the pair of comb-shaped electrodes 61 in which the Cr film was arranged on the ITO film formed on the glass substrate 60 having the stepped form.

A resist 46b was applied onto the substrate manufactured as described above by using a spin coater so that the height of the resist was constant. After performing natural drying for 1 minute, the prebaking (95°C, 3 minutes) was performed by using a hot plate. An epoxy-based negative type resist was used as the resist. Subsequently, by using a photomask for exposure on which a pattern aligned in an array form was depicted, the resist 46b was exposed by means of a UV exposure apparatus in a state where the micropores were positionally adjusted on the comb-shaped electrodes, followed by being developed with a developing solution. After the development, the exposed Cr film was exfoliated by means of 30% ceric ammonium nitrate solution so that ITO 37 was exposed at the bottom surface of the holding hole. After that, the postbaking (180°C, 30 minutes) was performed by using a hot plate to cause the curing of the resist, and thereby to form an insulator 46c and manufacture a comb-shaped electrode substrate 62 integrated with the holding unit (stack of the insulator film and the light shielding film) formed with the plurality of holding portions.

The spacer 3 was stacked and adhered under pressure on the holding unit on the comb-shaped electrode substrate manufactured as described above. A power source (signal generator) was connected to both of the pair of electrodes for constituting the comb-shaped electrodes via conductive lines 10. The biological sample can be also immobilized and analyzed by using the immobilizing apparatus configured as described above, similarly to Example 2.

### PARTS LIST

- 1:: upper electrode substrate
- 2:: lower electrode substrate
- 3:: spacer
- 4, 46c:: insulator
- 5:: holding portion (through-hole)
- 5':: diameter-expanded recess
- 6:: main body
- 7:: light shielding member
- 8:: introducing port
- 9:: discharge port
- 10:: conductive line
- 11:: AC power source
- 12:: electric flux line
- 13:: biological sample
- 14:: dielectrophoretic force
- 15:: substance which binds to biological sample
- 16:: temperature-responsive high molecular weight compound
- 17:: water-insoluble liquid
- 18:: abnormal cell
- 19:: microscope
- 20:: biological sample collecting means
- 21, 37:: ITO
- 22, 60:: glass
- 23, 38:: Cr
- 24, 40, 46, 46a, 46b:: resist
- 25, 39, 41:: photomask for exposure
- 26, 42:: exposure
- 27, 43, 47:: developing solution
- 28, 49:: 30% ceric ammonium nitrate solution
- 29:: electrode substrate
- 31:: one electrode
- 32:: the other electrode
- 33:: cell
- 48:: ITO etching solution
- 50:: accommodating unit

## Claims

1. A biological sample immobilizing apparatus comprising:
a holding unit which has a holding portion for holding a biological sample;
a power source which is capable of applying an AC voltage; and
a pair of electrodes which allows a dielectrophoretic force to act on said biological sample in a given dielectrophoretic space by means of said AC voltage applied from said power source in order to move said biological sample to said holding portion
wherein each of said pair of electrodes is arranged so that said dielectrophoretic force can act on said biological sample in said given dielectrophoretic space from the side of a common electrode installation position positioned on the side of said holding unit with respect to said given dielectrophoretic space.

2. The biological sample immobilizing apparatus according to claim 1,
wherein said holding unit has at least two of said holding portions; and
wherein electrode installation positions, at which respective electrodes of said pair of electrodes are arranged, are positioned on a common flat surface which defines respective internal spaces of said at least two holding portions possessed by said holding unit.

3. The biological sample immobilizing apparatus according to claim 2,
wherein one electrode of said pair of electrodes is arranged to be capable of making contact with said biological sample in some holding portion(s) of said at least two holding portions, and
wherein the other electrode of said pair of electrodes is arranged to be capable of making contact with said biological sample in the remaining holding portion(s) of said at least two holding portions.

4. The biological sample immobilizing apparatus according to claim 3,
wherein said holding portions each have an opening through which said biological sample enters from said given dielectrophoretic space,
wherein said one electrode is arranged at a bottom portion of said holding portion opposed to said opening in said some holding portion(s), and
wherein said other electrode is arranged at a bottom portion of said holding portion opposed to said opening in said remaining holding portion(s).

5. The biological sample immobilizing apparatus according to any one of claims 2 to 4,
wherein said pair of electrodes comprises:
a first electrode which has a plurality of small electrode portions and which is constructed by connecting said small electrode portions; and
a second electrode which is independent from said first electrode, which has a plurality of small electrode portions, and which is constructed by connecting said small electrode portions, and
wherein at least some of said plurality of small electrode portions of said first electrode and at least some of said plurality of small electrode portions of said second electrode are alternately arranged while keeping a given distance between the electrodes on said common flat surface.

6. The biological sample immobilizing apparatus according to any one of claims 2 to 4,
wherein said pair of electrodes comprises:
a first electrode which has a continuous line form; and
a second electrode which is independent from said first electrode and which has a continuous line form, and
wherein said first electrode and said second electrode are arranged on said common flat surface while keeping a given distance between the electrodes in a length direction between both of said electrodes.

7. The biological sample immobilizing apparatus according to claim 6, wherein said first electrode and said second electrode each are arranged in a continuous curved line form or in a continuous polygonal line form on said common flat surface.

8. The biological sample immobilizing apparatus according to any one of claims 1 to 7, wherein one end of said holding unit is opened so that said biological sample immobilized to said holding unit can be collected.
